# EUROPÄISCHE PATENTSCHRIFT

(11) **EP 1 848 733 B1**
(45) Veröffentlichungstag und Bekanntmachung des Hinweises auf die Patenterteilung: **21.06.2017**
(21) Anmeldenummer: 06708066.3
(22) Anmeldetag: 07.02.2006
(51) Int. Cl.: C07K 14/37

(54) **NEUE HYDROPHOBINFUSIONSPROTEINE, DEREN HERSTELLUNG UND VERWENDUNG**
NOVEL HYDROPHOBIN FUSION PRODUCTS, PRODUCTION AND USE THEREOF
NOUVELLES PROTEINES DE FUSION D'HYDROPHOBINE, LEUR PRODUCTION ET LEUR UTILISATION

(30) Priorität: 07.02.2005 DE 102005005737; 17.02.2005 DE 102005007480
(43) Veröffentlichungstag der Anmeldung: 31.10.2007
(73) Patentinhaber: BASF SE, 67056 Ludwigshafen am Rhein (DE)
(72) Erfinder: SUBKOWSKI, Thomas, 68526 Ladenburg (DE); KAROS, Marvin, 68723 Schwetzingen (DE); LEMAIRE, Hans-Georg, 67117 Limburgerhof (DE); BARG, Heiko, 67346 Speyer (DE); BOLLSCHWEILER, Claus, 69118 Heidelberg (DE)
(74) Vertreter: BASF IP Association
(86) Internationale Anmeldenummer: PCT/EP2006/050719
(87) Internationale Veröffentlichungsnummer: WO 2006/082251

(56) Entgegenhaltungen:
- WO-A-00/58342
- WO-A-01/57528
- LINDER MARKUS ET AL: "Surface adhesion of fusion proteins containing the hydrophobins HFBI and HFBII from Trichoderma reesei" PROTEIN SCIENCE, Bd. 11, Nr. 9, September 2002 (2002-09), Seiten 2257-2266, XP002386767 ISSN: 0961-8368
- STRINGER M A ET AL: "DEWA ENCODES A FUNGAL HYDROPHOBIN COMPONENT OF THE ASPERGILLUS SPORE WALL" MOLECULAR MICROBIOLOGY, BLACKWELL SCIENTIFIC, OXFORD, GB, Bd. 16, Nr. 1, 1995, Seiten 33-44, XP000770242 ISSN: 0950-382X -& DATABASE UniProt 1. Oktober 1996 (1996-10-01), XP002386772 gefunden im EBI Database accession no. P52750
- BELITSKY BORIS R: "Physical and enzymological interaction of Bacillus subtilis proteins required for de novo pyridoxal 5'-phosphate biosynthesis." JOURNAL OF BACTERIOLOGY, Bd. 186, Nr. 4, Februar 2004 (2004-02), Seiten 1191-1196, XP002386768 ISSN: 0021-9193 -& DATABASE UniProt 1. Oktober 1994 (1994-10-01), XP002386773 gefunden im EBI Database accession no. P37527 -& DATABASE UniProt 1. Oktober 1994 (1994-10-01), XP002386774 gefunden im EBI Database accession no. P37528
- WOESTEN H A B: "HYDROPHOBINS: MULTIPURPOSE PROTEINS" ANNUAL REVIEW OF MICROBIOLOGY, ANNUAL REVIEWS INC., PALO ALTO, CA, US, Bd. 55, 2001, Seiten 625-646, XP008003420 ISSN: 0066-4227

### SEQUENCE LISTING

<110> BASF Aktiengesellschaft
<120> Rekombinante Herstellung von Hydrophobinen
<130> AE 20040837
<160> 24
<170> PatentIn version 3.1
<210> 1
   <211> 405
   <212> DNA
   <213> basf-dewA
<220>
   <221> CDS
   <222> (1)..(405)
   <223>
<400> 1
   atg cgc ttc atc gtc tct ctc ctc gcc ttc act gcc gcg gcc acc gcg        48
   Met Arg Phe Ile Val Ser Leu Leu Ala Phe Thr Ala Ala Ala Thr Ala
   1               5                   10                  15
   acc gcc ctc ccg gcc tct gcc gca aag aac gcg aag ctg gcc acc tcg        96
   Thr Ala Leu Pro Ala Ser Ala Ala Lys Asn Ala Lys Leu Ala Thr Ser
               20                  25                  30
   gcg gcc ttc gcc aag cag gct gaa ggc acc acc tgc aat gtc ggc tcg       144
   Ala Ala Phe Ala Lys Gln Ala Glu Gly Thr Thr Cys Asn Val Gly Ser
           35                  40                  45
   atc gct tgc tgc aac tcc ccc gct gag acc aac aac gac agt ctg ttg       192
   Ile Ala Cys Cys Asn Ser Pro Ala Glu Thr Asn Asn Asp Ser Leu Leu
       50                  55                  60
   agc ggt ctg ctc ggt gct ggc ctt ctc aac ggg ctc tcg ggc aac act       240
   Ser Gly Leu Leu Gly Ala Gly Leu Leu Asn Gly Leu Ser Gly Asn Thr
   65                  70                  75                  80
   ggc agc gcc tgc gcc aag gcg agc ttg att gac cag ctg ggt ctg ctc       288
   Gly Ser Ala Cys Ala Lys Ala Ser Leu Ile Asp Gln Leu Gly Leu Leu
                   85                  90                  95
   gct ctc gtc gac cac act gag gaa ggc ccc gtc tgc aag aac atc gtc       336
   Ala Leu Val Asp His Thr Glu Glu Gly Pro Val Cys Lys Asn Ile Val
               100                 105                 110
   gct tgc tgc cct gag gga acc acc aac tgt gtt gcc gtc gac aac gct       384
   Ala Cys Cys Pro Glu Gly Thr Thr Asn Cys Val Ala Val Asp Asn Ala
           115                 120                 125
   ggc gct ggt acc aag gct gag                                           405
   Gly Ala Gly Thr Lys Ala Glu
       130                 135
<210> 2
   <211> 135
   <212> PRT
   <213> basf-dewA
<400> 2
   Met Arg Phe Ile Val Ser Leu Leu Ala Phe Thr Ala Ala Ala Thr Ala
   1               5                   10                  15
   Thr Ala Leu Pro Ala Ser Ala Ala Lys Asn Ala Lys Leu Ala Thr Ser
               20                  25                  30
   Ala Ala Phe Ala Lys Gln Ala Glu Gly Thr Thr Cys Asn Val Gly Ser
           35                  40                  45
   Ile Ala Cys Cys Asn Ser Pro Ala Glu Thr Asn Asn Asp Ser Leu Leu
       50                  55                  60
   Ser Gly Leu Leu Gly Ala Gly Leu Leu Asn Gly Leu Ser Gly Asn Thr
   65                  70                  75                  80
   Gly Ser Ala Cys Ala Lys Ala Ser Leu Ile Asp Gln Leu Gly Leu Leu
                   85                  90                  95
   Ala Leu Val Asp His Thr Glu Glu Gly Pro Val Cys Lys Asn Ile Val
               100                 105                 110
   Ala Cys Cys Pro Glu Gly Thr Thr Asn Cys Val Ala Val Asp Asn Ala
           115                 120                 125
   Gly Ala Gly Thr Lys Ala Glu
       130                 135
<210> 3
   <211> 471
   <212> DNA
   <213> basf-rodA
<220>
   <221> CDS
   <222> (1)..(471)
   <223>
<400> 3
   atg aag ttc tcc att gct gcc gct gtc gtt gct ttc gcc gcc tcc gtc        48
   Met Lys Phe Ser Ile Ala Ala Ala Val Val Ala Phe Ala Ala Ser Val
   1               5                   10                  15
   gcg gcc ctc cct cct gcc cat gat tcc cag ttc gct ggc aat ggt gtt        96
   Ala Ala Leu Pro Pro Ala His Asp Ser Gln Phe Ala Gly Asn Gly Val
               20                  25                  30
   ggc aac aag ggc aac agc aac gtc aag ttc cct gtc ccc gaa aac gtg       144
   Gly Asn Lys Gly Asn Ser Asn Val Lys Phe Pro Val Pro Glu Asn Val
           35                  40                  45
   acc gtc aag cag gcc tcc gac aag tgc ggt gac cag gcc cag ctc tct       192
   Thr Val Lys Gln Ala Ser Asp Lys Cys Gly Asp Gln Ala Gln Leu Ser
       50                  55                  60
   tgc tgc aac aag gcc acg tac gcc ggt gac acc aca acc gtt gat gag       240
   Cys Cys Asn Lys Ala Thr Tyr Ala Gly Asp Thr Thr Thr Val Asp Glu
   65                  70                  75                  80
   ggt ctt ctg tct ggt gcc ctc agc ggc ctc atc ggc gcc ggg tct ggt       288
   Gly Leu Leu Ser Gly Ala Leu Ser Gly Leu Ile Gly Ala Gly Ser Gly
                   85                  90                  95
   gcc gaa ggt ctt ggt ctc ttc gat cag tgc tcc aag ctt gat gtt gct       336
   Ala Glu Gly Leu Gly Leu Phe Asp Gln Cys Ser Lys Leu Asp Val Ala
               100                 105                 110
   gtc ctc att ggc atc caa gat ctt gtc aac cag aag tgc aag caa aac       384
   Val Leu Ile Gly Ile Gln Asp Leu Val Asn Gln Lys Cys Lys Gln Asn
           115                 120                 125
   att gcc tgc tgc cag aac tcc ccc tcc agc gcg gat ggc aac ctt att       432
   Ile Ala Cys Cys Gln Asn Ser Pro Ser Ser Ala Asp Gly Asn Leu Ile
       130                 135                 140
   ggt gtc ggt ctc cct tgc gtt gcc ctt ggc tcc atc ctc                   471
   Gly Val Gly Leu Pro Cys Val Ala Leu Gly Ser Ile Leu
   145                 150                 155
<210> 4
   <211> 157
   <212> PRT
   <213> basf-rodA
<400> 4
   Met Lys Phe Ser Ile Ala Ala Ala Val Val Ala Phe Ala Ala Ser Val
   1               5                   10                  15
   Ala Ala Leu Pro Pro Ala His Asp Ser Gln Phe Ala Gly Asn Gly Val
               20                  25                  30
   Gly Asn Lys Gly Asn Ser Asn Val Lys Phe Pro Val Pro Glu Asn Val
           35                  40                  45
   Thr Val Lys Gln Ala Ser Asp Lys Cys Gly Asp Gln Ala Gln Leu Ser
       50                  55                  60
   Cys Cys Asn Lys Ala Thr Tyr Ala Gly Asp Thr Thr Thr Val Asp Glu
   65                  70                  75                  80
   Gly Leu Leu Ser Gly Ala Leu Ser Gly Leu Ile Gly Ala Gly Ser Gly
                   85                  90                  95
   Ala Glu Gly Leu Gly Leu Phe Asp Gln Cys Ser Lys Leu Asp Val Ala
               100                 105                 110
   Val Leu Ile Gly Ile Gln Asp Leu Val Asn Gln Lys Cys Lys Gln Asn
           115                 120                 125
   Ile Ala Cys Cys Gln Asn Ser Pro Ser Ser Ala Asp Gly Asn Leu Ile
       130                 135                 140
   Gly Val Gly Leu Pro Cys Val Ala Leu Gly Ser Ile Leu
   145                 150                 155
<210> 5
   <211> 336
   <212> DNA
   <213> basf-HypA
<220>
   <221> CDS
   <222> (1)..(336)
   <223>
<400> 5
   atg atc tct cgc gtc ctt gtc gct gct ctc gtc gct ctc ccc gct ctt       48
   Met Ile Ser Arg Val Leu Val Ala Ala Leu Val Ala Leu Pro Ala Leu
   1               5                   10                  15
   gtt act gca act cct gct ccc gga aag cct aaa gcc agc agt cag tgc       96
   Val Thr Ala Thr Pro Ala Pro Gly Lys Pro Lys Ala Ser Ser Gln Cys
               20                  25                  30
   gac gtc ggt gaa atc cat tgc tgt gac act cag cag act ccc gac cac      144
   Asp Val Gly Glu Ile His Cys Cys Asp Thr Gln Gln Thr Pro Asp His
           35                  40                  45
   acc agc gcc gcc gcg tct ggt ttg ctt ggt gtt ccc atc aac ctt ggt      192
   Thr Ser Ala Ala Ala Ser Gly Leu Leu Gly Val Pro Ile Asn Leu Gly
       50                  55                  60
   gct ttc ctc ggt ttc gac tgt acc ccc att tcc gtc ctt ggc gtc ggt      240
   Ala Phe Leu Gly Phe Asp Cys Thr Pro Ile Ser Val Leu Gly Val Gly
   65                  70                  75                  80
   ggc aac aac tgt gct gct cag cct gtc tgc tgc aca gga aat caa ttc      288
   Gly Asn Asn Cys Ala Ala Gln Pro Val Cys Cys Thr Gly Asn Gln Phe
                   85                  90                  95
   acc gca ttg att aac gct ctt gac tgc tct cct gtc aat gtc aac ctc      336
   Thr Ala Leu Ile Asn Ala Leu Asp Cys Ser Pro Val Asn Val Asn Leu
               100                 105                 110
<210> 6
   <211> 112
   <212> PRT
   <213> basf-HypA
<400> 6
   Met Ile Ser Arg Val Leu Val Ala Ala Leu Val Ala Leu Pro Ala Leu
   1               5                   10                  15
   Val Thr Ala Thr Pro Ala Pro Gly Lys Pro Lys Ala Ser Ser Gln Cys
               20                  25                  30
   Asp Val Gly Glu Ile His Cys Cys Asp Thr Gln Gln Thr Pro Asp His
           35                  40                  45
   Thr Ser Ala Ala Ala Ser Gly Leu Leu Gly Val Pro Ile Asn Leu Gly
       50                  55                  60
   Ala Phe Leu Gly Phe Asp Cys Thr Pro Ile Ser Val Leu Gly Val Gly
   65                  70                  75                  80
   Gly Asn Asn Cys Ala Ala Gln Pro Val Cys Cys Thr Gly Asn Gln Phe
                   85                  90                  95
   Thr Ala Leu Ile Asn Ala Leu Asp Cys Ser Pro Val Asn Val Asn Leu
               100                 105                 110
<210> 7
   <211> 357
   <212> DNA
   <213> basf-HypB
<220>
   <221> CDS
   <222> (1)..(357)
   <223>
<400> 7
   atg gtc agc acg ttc atc act gtc gca aag acc ctt ctc gtc gcg ctc      48
   Met Val Ser Thr Phe Ile Thr Val Ala Lys Thr Leu Leu Val Ala Leu
   1               5                   10                  15
   ctc ttc gtc aat atc aat atc gtc gtt ggt act gca act acc ggc aag      96
   Leu Phe Val Asn Ile Asn Ile Val Val Gly Thr Ala Thr Thr Gly Lys
               20                  25                  30
   cat tgt agc acc ggt cct atc gag tgc tgc aag cag gtc atg gat tct      144
   His Cys Ser Thr Gly Pro Ile Glu Cys Cys Lys Gln Val Met Asp Ser
           35                  40                  45
   aag agc cct cag gct acg gag ctt ctt acg aag aat ggc ctt ggc ctg      192
   Lys Ser Pro Gln Ala Thr Glu Leu Leu Thr Lys Asn Gly Leu Gly Leu
       50                  55                  60
   ggt gtc ctt gct ggc gtg aag ggt ctt gtt ggc gcg aat tgc agc cct      240
   Gly Val Leu Ala Gly Val Lys Gly Leu Val Gly Ala Asn Cys Ser Pro
   65                  70                  75                  80
   atc acg gca att ggt att ggc tcc ggc agc caa tgc tct ggc cag acc      288
   Ile Thr Ala Ile Gly Ile Gly Ser Gly Ser Gln Cys Ser Gly Gln Thr
                   85                  90                  95
   gtt tgc tgc cag aat aat aat ttc aac ggt gtt gtc gct att ggt tgc      336
   Val Cys Cys Gln Asn Asn Asn Phe Asn Gly Val Val Ala Ile Gly Cys
               100                 105                 110
   act ccc att aat gcc aat gtg                                          357
   Thr Pro Ile Asn Ala Asn Val
           115
<210> 8
   <211> 119
   <212> PRT
   <213> basf-HypB
<400> 8
   Met Val Ser Thr Phe Ile Thr Val Ala Lys Thr Leu Leu Val Ala Leu
   1               5                   10                  15
   Leu Phe Val Asn Ile Asn Ile Val Val Gly Thr Ala Thr Thr Gly Lys
               20                  25                  30
   His Cys Ser Thr Gly Pro Ile Glu Cys Cys Lys Gln Val Met Asp Ser
               35                  40                  45
   Lys Ser Pro Gln Ala Thr Glu Leu Leu Thr Lys Asn Gly Leu Gly Leu
           50                  55                  60
   Gly Val Leu Ala Gly Val Lys Gly Leu Val Gly Ala Asn Cys Ser Pro
   65                  70                  75                  80
   Ile Thr Ala Ile Gly Ile Gly Ser Gly Ser Gln Cys Ser Gly Gln Thr
                   85                  90                  95
   Val Cys Cys Gln Asn Asn Asn Phe Asn Gly Val Val Ala Ile Gly Cys
               100                 105                 110
   Thr Pro Ile Asn Ala Asn Val
               115
<210> 9
   <211> 408
   <212> DNA
   <213> basf-sc3
<220>
   <221> CDS
   <222> (1)..(408)
   <223>
<400> 9
   atg ttc gcc cgt ctc ccc gtc gtg ttc ctc tac gcc ttc gtc gcg ttc      48
   Met Phe Ala Arg Leu Pro Val Val Phe Leu Tyr Ala Phe Val Ala Phe
   1               5                   10                  15
   ggc gcc ctc gtc gct gcc ctc cca ggt ggc cac ccg ggc acg acc acg      96
   Gly Ala Leu Val Ala Ala Leu Pro Gly Gly His Pro Gly Thr Thr Thr
               20                  25                  30
   ccg ccg gtt acg acg acg gtg acg gtg acc acg ccg ccc tcg acg acg     144
   Pro Pro Val Thr Thr Thr Val Thr Val Thr Thr Pro Pro Ser Thr Thr
           35                  40                  45
   acc atc gcc gcc ggt ggc acg tgt act acg ggg tcg ctc tct tgc tgc     192
   Thr Ile Ala Ala Gly Gly Thr Cys Thr Thr Gly Ser Leu Ser Cys Cys
       50                  55                  60
   aac cag gtt caa tcg gcg agc agc agc cct gtt acc gcc ctc ctc ggc     240
   Asn Gln Val Gln Ser Ala Ser Ser Ser Pro Val Thr Ala Leu Leu Gly
   65                  70                  75                  80
   ctg ctc ggc att gtc ctc agc gac ctc aac gtt ctc gtt ggc atc agc     288
   Leu Leu Gly Ile Val Leu Ser Asp Leu Asn Val Leu Val Gly Ile Ser
                   85                  90                  95
   tgc tct ccc ctc act gtc atc ggt gtc gga ggc agc ggc tgt tcg gcg      336
   Cys Ser Pro Leu Thr Val Ile Gly Val Gly Gly Ser Gly Cys Ser Ala
               100                 105                 110
   cag acc gtc tgc tgc gaa aac acc caa ttc aac ggg ctg atc aac atc      384
   Gln Thr Val Cys Cys Glu Asn Thr Gln Phe Asn Gly Leu Ile Asn Ile
           115                 120                 125
   ggt tgc acc ccc atc aac atc ctc                                      408
   Gly Cys Thr Pro Ile Asn Ile Leu
       130                 135
<210> 10
   <211> 136
   <212> PRT
   <213> basf-sc3
<400> 10
   Met Phe Ala Arg Leu Pro Val Val Phe Leu Tyr Ala Phe Val Ala Phe
   1               5                   10                  15
   Gly Ala Leu Val Ala Ala Leu Pro Gly Gly His Pro Gly Thr Thr Thr
               20                  25                  30
   Pro Pro Val Thr Thr Thr Val Thr Val Thr Thr Pro Pro Ser Thr Thr
           35                  40                  45
   Thr Ile Ala Ala Gly Gly Thr Cys Thr Thr Gly Ser Leu Ser Cys Cys
       50                  55                  60
   Asn Gln Val Gln Ser Ala Ser Ser Ser Pro Val Thr Ala Leu Leu Gly
   65                  70                  75                  80
   Leu Leu Gly Ile Val Leu Ser Asp Leu Asn Val Leu Val Gly Ile Ser
                   85                  90                  95
   Cys Ser Pro Leu Thr Val Ile Gly Val Gly Gly Ser Gly Cys Ser Ala
               100                 105                 110
   Gln Thr Val Cys Cys Glu Asn Thr Gln Phe Asn Gly Leu Ile Asn Ile
           115                 120                 125
   Gly Cys Thr Pro Ile Asn Ile Leu
       130                 135
<210> 11
   <211> 483
   <212> DNA
   <213> basf-BASF1
<220>
   <221> CDS
   <222> (1)..(483)
   <223>
<400> 11
   atg aag ttc tcc gtc tcc gcc gcc gtc ctc gcc ttc gcc gcc tcc gtc       48
   Met Lys Phe Ser Val Ser Ala Ala Val Leu Ala Phe Ala Ala Ser Val
   1               5                   10                  15
   gcc gcc ctc cct cag cac gac tcc gcc gcc ggc aac ggc aac ggc gtc       96
   Ala Ala Leu Pro Gln His Asp Ser Ala Ala Gly Asn Gly Asn Gly Val
               20                  25                  30
   ggc aac aag ttc cct gtc cct gac gac gtc acc gtc aag cag gcc acc      144
   Gly Asn Lys Phe Pro Val Pro Asp Asp Val Thr Val Lys Gln Ala Thr
           35                  40                  45
   gac aag tgc ggc gac cag gcc cag ctc tcc tgc tgc aac aag gcc acc      192
   Asp Lys Cys Gly Asp Gln Ala Gln Leu Ser Cys Cys Asn Lys Ala Thr
       50                  55                  60
   tac gcc ggc gac gtc ctc acc gac atc gac gag ggc atc ctc gcc ggc      240
   Tyr Ala Gly Asp Val Leu Thr Asp Ile Asp Glu Gly Ile Leu Ala Gly
   65                  70                  75                  80
   ctc ctc aag aac ctc atc ggc ggc ggc tcc ggc tcc gag ggc ctc ggc      288
   Leu Leu Lys Asn Leu Ile Gly Gly Gly Ser Gly Ser Glu Gly Leu Gly
                   85                  90                  95
   ctc ttc gac cag tgc gtc aag ctc gac ctc cag atc tcc gtc atc ggc      336
   Leu Phe Asp Gln Cys Val Lys Leu Asp Leu Gln Ile Ser Val Ile Gly
               100                 105                 110
   atc cct atc cag gac ctc ctc aac cag gtc aac aag cag tgc aag cag      384
   Ile Pro Ile Gln Asp Leu Leu Asn Gln Val Asn Lys Gln Cys Lys Gln
           115                 120                 125
   aac atc gcc tgc tgc cag aac tcc cct tcc gac gcc acc ggc tcc ctc      432
   Asn Ile Ala Cys Cys Gln Asn Ser Pro Ser Asp Ala Thr Gly Ser Leu
       130                 135                 140
   gtc aac ctc ggc ctc ggc aac cct tgc atc cct gtc tcc ctc ctc cat      480
   Val Asn Leu Gly Leu Gly Asn Pro Cys Ile Pro Val Ser Leu Leu His
   145                 150                 155                 160
   atg                                                                  483
   Met
<210> 12
   <211> 161
   <212> PRT
   <213> basf-BASF1
<400> 12
   Met Lys Phe Ser Val Ser Ala Ala Val Leu Ala Phe Ala Ala Ser Val
   1               5                   10                  15
   Ala Ala Leu Pro Gln His Asp Ser Ala Ala Gly Asn Gly Asn Gly Val
               20                  25                  30
   Gly Asn Lys Phe Pro Val Pro Asp Asp Val Thr Val Lys Gln Ala Thr
           35                  40                  45
   Asp Lys Cys Gly Asp Gln Ala Gln Leu Ser Cys Cys Asn Lys Ala Thr
       50                  55                  60
   Tyr Ala Gly Asp Val Leu Thr Asp Ile Asp Glu Gly Ile Leu Ala Gly
   65                  70                  75                  80
   Leu Leu Lys Asn Leu Ile Gly Gly Gly Ser Gly Ser Glu Gly Leu Gly
                   85                  90                  95
   Leu Phe Asp Gln Cys Val Lys Leu Asp Leu Gln Ile Ser Val Ile Gly
               100                 105                 110
   Ile Pro Ile Gln Asp Leu Leu Asn Gln Val Asn Lys Gln Cys Lys Gln
           115                 120                 125
   Asn Ile Ala Cys Cys Gln Asn Ser Pro Ser Asp Ala Thr Gly Ser Leu
       130                 135                 140
   Val Asn Leu Gly Leu Gly Asn Pro Cys Ile Pro Val Ser Leu Leu His
   145                 150                 155                 160
   Met
<210> 13
   <211> 465
   <212> DNA
   <213> basf-BASF2
<220>
   <221> CDS
   <222> (1)..(465)
   <223>
<400> 13
   atg aag ttc tcc gtc tcc gcc gcc gtc ctc gcc ttc gcc gcc tcc gtc        48
   Met Lys Phe Ser Val Ser Ala Ala Val Leu Ala Phe Ala Ala Ser Val
   1               5                   10                  15
   gcc gcc ctc cct cag cac gac tcc gcc gcc ggc aac ggc aac ggc gtc        96
   Ala Ala Leu Pro Gln His Asp Ser Ala Ala Gly Asn Gly Asn Gly Val
               20                  25                  30
   ggc aac aag ttc cct gtc cct gac gac gtc acc gtc aag cag gcc acc       144
   Gly Asn Lys Phe Pro Val Pro Asp Asp Val Thr Val Lys Gln Ala Thr
           35                  40                  45
   gac aag tgc ggc gac cag gcc cag ctc tcc tgc tgc aac aag gcc acc       192
   Asp Lys Cys Gly Asp Gln Ala Gln Leu Ser Cys Cys Asn Lys Ala Thr
       50                  55                  60
   tac gcc ggc gac gtc acc gac atc gac gag ggc atc ctc gcc ggc ctc       240
   Tyr Ala Gly Asp Val Thr Asp Ile Asp Glu Gly Ile Leu Ala Gly Leu
   65                  70                  75                  80
   ctc aag aac ctc atc ggc ggc ggc tcc ggc tcc gag ggc ctc ggc ctc       288
   Leu Lys Asn Leu Ile Gly Gly Gly Ser Gly Ser Glu Gly Leu Gly Leu
                   85                  90                  95
   ttc gac cag tgc gtc aag ctc gac ctc cag atc tcc gtc atc ggc atc       336
   Phe Asp Gln Cys Val Lys Leu Asp Leu Gln Ile Ser Val Ile Gly Ile
               100                 105                 110
   cct atc cag gac ctc ctc aac cag cag tgc aag cag aac atc gcc tgc       384
   Pro Ile Gln Asp Leu Leu Asn Gln Gln Cys Lys Gln Asn Ile Ala Cys
           115                 120                 125
   tgc cag aac tcc cct tcc gac gcc acc ggc tcc ctc gtc aac ctc ggc       432
   Cys Gln Asn Ser Pro Ser Asp Ala Thr Gly Ser Leu Val Asn Leu Gly
       130                 135                 140
   aac cct tgc atc cct gtc tcc ctc ctc cat atg                           465
   Asn Pro Cys Ile Pro Val Ser Leu Leu His Met
   145                 150                 155
<210> 14
   <211> 155
   <212> PRT
   <213> basf-BASF2
<400> 14
   Met Lys Phe Ser Val Ser Ala Ala Val Leu Ala Phe Ala Ala Ser Val
   1               5                   10                  15
   Ala Ala Leu Pro Gln His Asp Ser Ala Ala Gly Asn Gly Asn Gly Val
               20                  25                  30
   Gly Asn Lys Phe Pro Val Pro Asp Asp Val Thr Val Lys Gln Ala Thr
           35                  40                  45
   Asp Lys Cys Gly Asp Gln Ala Gln Leu Ser Cys Cys Asn Lys Ala Thr
       50                  55                  60
   Tyr Ala Gly Asp Val Thr Asp Ile Asp Glu Gly Ile Leu Ala Gly Leu
   65                  70                  75                  80
   Leu Lys Asn Leu Ile Gly Gly Gly Ser Gly Ser Glu Gly Leu Gly Leu
                   85                  90                  95
   Phe Asp Gln Cys Val Lys Leu Asp Leu Gln Ile Ser Val Ile Gly Ile
               100                 105                 110
   Pro Ile Gln Asp Leu Leu Asn Gln Gln Cys Lys Gln Asn Ile Ala Cys
           115                 120                 125
   Cys Gln Asn Ser Pro Ser Asp Ala Thr Gly Ser Leu Val Asn Leu Gly
       130                 135                 140
   Asn Pro Cys Ile Pro Val Ser Leu Leu His Met
   145                 150                 155
<210> 15
   <211> 882
   <212> DNA
   <213> basf-yaad
<220>
   <221> CDS
   <222> (1)..(882)
   <223>
<400> 15
   atg gct caa aca ggt act gaa cgt gta aaa cgc gga atg gca gaa atg       48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa       96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg      144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct      192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca      240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg      288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa      336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc      384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct      432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct      480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg      528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct      576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt      624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg      672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa      720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act      768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt      816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt      864
   Thr Ala Met Lys Gly Ile Glu Ile Ser Asn Leu Leu Pro Glu Gln Arg
           275                 280                 285
   atg caa gaa cgc ggc tgg                                              882
   Met Gln Glu Arg Gly Trp
       290
<210> 16
   <211> 294
   <212> PRT
   <213> basf-yaad
<400> 16
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp
       290
<210> 17
   <211> 591
   <212> DNA
   <213> basf-yaae
<220>
   <221> CDS
   <222> (1)..(591)
   <223>
<400> 17
   atg gga tta aca ata ggt gta cta gga ctt caa gga gca gtt aga gag      48
   Met Gly Leu Thr Ile Gly Val Leu Gly Leu Gln Gly Ala Val Arg Glu
   1               5                   10                  15
   cac atc cat gcg att gaa gca tgc ggc gcg gct ggt ctt gtc gta aaa      96
   His Ile His Ala Ile Glu Ala Cys Gly Ala Ala Gly Leu Val Val Lys
               20                  25                  30
   cgt ccg gag cag ctg aac gaa gtt gac ggg ttg att ttg ccg ggc ggt     144
   Arg Pro Glu Gln Leu Asn Glu Val Asp Gly Leu Ile Leu Pro Gly Gly
           35                  40                  45
   gag agc acg acg atg cgc cgt ttg atc gat acg tat caa ttc atg gag     192
   Glu Ser Thr Thr Met Arg Arg Leu Ile Asp Thr Tyr Gln Phe Met Glu
       50                  55                  60
   ccg ctt cgt gaa ttc gct gct cag ggc aaa ccg atg ttt gga aca tgt     240
   Pro Leu Arg Glu Phe Ala Ala Gln Gly Lys Pro Met Phe Gly Thr Cys
   65                  70                  75                  80
   gcc gga tta att ata tta gca aaa gaa att gcc ggt tca gat aat cct     288
   Ala Gly Leu Ile Ile Leu Ala Lys Glu Ile Ala Gly Ser Asp Asn Pro
                   85                  90                  95
   cat tta ggt ctt ctg aat gtg gtt gta gaa cgt aat tca ttt ggc cgg     336
   His Leu Gly Leu Leu Asn Val Val Val Glu Arg Asn Ser Phe Gly Arg
               100                 105                 110
   cag gtt gac agc ttt gaa gct gat tta aca att aaa ggc ttg gac gag     384
   Gln Val Asp Ser Phe Glu Ala Asp Leu Thr Ile Lys Gly Leu Asp Glu
           115                 120                 125
   cct ttt act ggg gta ttc atc cgt gct ccg cat att tta gaa gct ggt     432
   Pro Phe Thr Gly Val Phe Ile Arg Ala Pro His Ile Leu Glu Ala Gly
       130                 135                 140
   gaa aat gtt gaa gtt cta tcg gag cat aat ggt cgt att gta gcc gcg     480
   Glu Asn Val Glu Val Leu Ser Glu His Asn Gly Arg Ile Val Ala Ala
   145                 150                 155                 160
   aaa cag ggg caa ttc ctt ggc tgc tca ttc cat ccg gag ctg aca gaa      528
   Lys Gln Gly Gln Phe Leu Gly Cys Ser Phe His Pro Glu Leu Thr Glu
                   165                 170                 175
   gat cac cga gtg acg cag ctg ttt gtt gaa atg gtt gag gaa tat aag      576
   Asp His Arg Val Thr Gln Leu Phe Val Glu Met Val Glu Glu Tyr Lys
               180                 185                 190
   caa aag gca ctt gta                                                  591
   Gln Lys Ala Leu Val
           195
<210> 18
   <211> 197
   <212> PRT
   <213> basf-yaae
<400> 18
   Met Gly Leu Thr Ile Gly Val Leu Gly Leu Gln Gly Ala Val Arg Glu
   1               5                   10                  15
   His Ile His Ala Ile Glu Ala Cys Gly Ala Ala Gly Leu Val Val Lys
               20                  25                  30
   Arg Pro Glu Gln Leu Asn Glu Val Asp Gly Leu Ile Leu Pro Gly Gly
           35                  40                  45
   Glu Ser Thr Thr Met Arg Arg Leu Ile Asp Thr Tyr Gln Phe Met Glu
       50                  55                  60
   Pro Leu Arg Glu Phe Ala Ala Gln Gly Lys Pro Met Phe Gly Thr Cys
   65                  70                  75                  80
   Ala Gly Leu Ile Ile Leu Ala Lys Glu Ile Ala Gly Ser Asp Asn Pro
                   85                  90                  95
   His Leu Gly Leu Leu Asn Val Val Val Glu Arg Asn Ser Phe Gly Arg
               100                 105                 110
   Gln Val Asp Ser Phe Glu Ala Asp Leu Thr Ile Lys Gly Leu Asp Glu
           115                 120                 125
   Pro Phe Thr Gly Val Phe Ile Arg Ala Pro His Ile Leu Glu Ala Gly
       130                 135                 140
   Glu Asn Val Glu Val Leu Ser Glu His Asn Gly Arg Ile Val Ala Ala
   145                 150                 155                 160
   Lys Gln Gly Gln Phe Leu Gly Cys Ser Phe His Pro Glu Leu Thr Glu
                   165                 170                 175
   Asp His Arg Val Thr Gln Leu Phe Val Glu Met Val Glu Glu Tyr Lys
               180                 185                 190
   Gln Lys Ala Leu Val
           195
<210> 19
   <211> 1329
   <212> DNA
   <213> basf-yaad-Xa-dewA-his
<220>
   <221> CDS
   <222> (1)..(1329)
   <223>
<400> 19
   atg gct caa aca ggt act gaa cgt gta aaa cgc gga atg gca gaa atg       48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa       96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg      144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct      192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca      240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg      288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa      336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc      384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct      432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct       480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg       528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct       576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt       624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg       672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa       720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act       768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt       816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt       864
   Thr Ala Met Lys Gly Ile Glu Ile Ser Asn Leu Leu Pro Glu Gln Arg
           275                 280                 285
   atg caa gaa cgc ggc tgg aga tcc att gaa ggc cgc atg cgc ttc atc       912
   Met Gln Glu Arg Gly Trp Arg Ser Ile Glu Gly Arg Met Arg Phe Ile
       290                 295                 300
   gtc tct ctc ctc gcc ttc act gcc gcg gcc acc gcg acc gcc ctc ccg       960
   Val Ser Leu Leu Ala Phe Thr Ala Ala Ala Thr Ala Thr Ala Leu Pro
   305                 310                 315                 320
   gcc tct gcc gca aag aac gcg aag ctg gcc acc tcg gcg gcc ttc gcc      1008
   Ala Ser Ala Ala Lys Asn Ala Lys Leu Ala Thr Ser Ala Ala Phe Ala
                   325                 330                 335
   aag cag gct gaa ggc acc acc tgc aat gtc ggc tcg atc gct tgc tgc      1056
   Lys Gln Ala Glu Gly Thr Thr Cys Asn Val Gly Ser Ile Ala Cys Cys
               340                 345                 350
   aac tcc ccc gct gag acc aac aac gac agt ctg ttg agc ggt ctg ctc      1104
   Asn Ser Pro Ala Glu Thr Asn Asn Asp Ser Leu Leu Ser Gly Leu Leu
           355                 360                 365
   ggt gct ggc ctt ctc aac ggg ctc tcg ggc aac act ggc agc gcc tgc      1152
   Gly Ala Gly Leu Leu Asn Gly Leu Ser Gly Asn Thr Gly Ser Ala Cys
       370                 375                 380
   gcc aag gcg agc ttg att gac cag ctg ggt ctg ctc gct ctc gtc gac      1200
   Ala Lys Ala Ser Leu Ile Asp Gln Leu Gly Leu Leu Ala Leu Val Asp
   385                 390                 395                 400
   cac act gag gaa ggc ccc gtc tgc aag aac atc gtc gct tgc tgc cct      1248
   His Thr Glu Glu Gly Pro Val Cys Lys Asn Ile Val Ala Cys Cys Pro
                   405                 410                 415
   gag gga acc acc aac tgt gtt gcc gtc gac aac gct ggc gct ggt acc      1296
   Glu Gly Thr Thr Asn Cys Val Ala Val Asp Asn Ala Gly Ala Gly Thr
               420                 425                 430
   aag gct gag gga tct cat cac cat cac cat cac                          1329
   Lys Ala Glu Gly Ser His His His His His His
           435                 440
<210> 20
   <211> 443
   <212> PRT
   <213> basf-yaad-Xa-dewA-his
<400> 20
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp Arg Ser Ile Glu Gly Arg Met Arg Phe Ile
       290                 295                 300
   Val Ser Leu Leu Ala Phe Thr Ala Ala Ala Thr Ala Thr Ala Leu Pro
   305                 310                 315                 320
   Ala Ser Ala Ala Lys Asn Ala Lys Leu Ala Thr Ser Ala Ala Phe Ala
                   325                 330                 335
   Lys Gln Ala Glu Gly Thr Thr Cys Asn Val Gly Ser Ile Ala Cys Cys
               340                 345                 350
   Asn Ser Pro Ala Glu Thr Asn Asn Asp Ser Leu Leu Ser Gly Leu Leu
           355                 360                 365
   Gly Ala Gly Leu Leu Asn Gly Leu Ser Gly Asn Thr Gly Ser Ala Cys
       370                 375                 380
   Ala Lys Ala Ser Leu Ile Asp Gln Leu Gly Leu Leu Ala Leu Val Asp
   385                 390                 395                 400
   His Thr Glu Glu Gly Pro Val Cys Lys Asn Ile Val Ala Cys Cys Pro
                   405                 410                 415
   Glu Gly Thr Thr Asn Cys Val Ala Val Asp Asn Ala Gly Ala Gly Thr
               420                 425                 430
   Lys Ala Glu Gly Ser His His His His His His
           435                 440
<210> 21
   <211> 1395
   <212> DNA
   <213> basf-yaad-Xa-rodA-his
<220>
   <221> CDS
   <222> (1)..(1395)
   <223>
<400> 21
   atg gct caa aca ggt act gaa cgt gta aaa cgc gga atg gca gaa atg      48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa      96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg      144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct      192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca      240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg      288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa      336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc      384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct      432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct      480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg      528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct      576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt      624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg      672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa      720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act      768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt      816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt      864
   Thr Ala Met Lys Gly Ile Glu Ile Ser Asn Leu Leu Pro Glu Gln Arg
           275                 280                 285
   atg caa gaa cgc ggc tgg aga tct att gaa ggc cgc atg aag ttc tcc      912
   Met Gln Glu Arg Gly Trp Arg Ser Ile Glu Gly Arg Met Lys Phe Ser
       290                 295                 300
   att gct gcc gct gtc gtt gct ttc gcc gcc tcc gtc gcg gcc ctc cct      960
   Ile Ala Ala Ala Val Val Ala Phe Ala Ala Ser Val Ala Ala Leu Pro
   305                 310                 315                 320
   cct gcc cat gat tcc cag ttc gct ggc aat ggt gtt ggc aac aag ggc     1008
   Pro Ala His Asp Ser Gln Phe Ala Gly Asn Gly Val Gly Asn Lys Gly
                   325                 330                 335
   aac agc aac gtc aag ttc cct gtc ccc gaa aac gtg acc gtc aag cag     1056
   Asn Ser Asn Val Lys Phe Pro Val Pro Glu Asn Val Thr Val Lys Gln
               340                 345                 350
   gcc tcc gac aag tgc ggt gac cag gcc cag ctc tct tgc tgc aac aag     1104
   Ala Ser Asp Lys Cys Gly Asp Gln Ala Gln Leu Ser Cys Cys Asn Lys
           355                 360                 365
   gcc acg tac gcc ggt gac acc aca acc gtt gat gag ggt ctt ctg tct     1152
   Ala Thr Tyr Ala Gly Asp Thr Thr Thr Val Asp Glu Gly Leu Leu Ser
       370                 375                 380
   ggt gcc ctc agc ggc ctc atc ggc gcc ggg tct ggt gcc gaa ggt ctt     1200
   Gly Ala Leu Ser Gly Leu Ile Gly Ala Gly Ser Gly Ala Glu Gly Leu
   385                 390                 395                 400
   ggt ctc ttc gat cag tgc tcc aag ctt gat gtt gct gtc ctc att ggc     1248
   Gly Leu Phe Asp Gln Cys Ser Lys Leu Asp Val Ala Val Leu Ile Gly
                   405                 410                 415
   atc caa gat ctt gtc aac cag aag tgc aag caa aac att gcc tgc tgc     1296
   Ile Gln Asp Leu Val Asn Gln Lys Cys Lys Gln Asn Ile Ala Cys Cys
               420                 425                 430
   cag aac tcc ccc tcc agc gcg gat ggc aac ctt att ggt gtc ggt ctc     1344
   Gln Asn Ser Pro Ser Ser Ala Asp Gly Asn Leu Ile Gly Val Gly Leu
           435                 440                 445
   cct tgc gtt gcc ctt ggc tcc atc ctc gga tct cat cac cat cac cat     1392
   Pro Cys Val Ala Leu Gly Ser Ile Leu Gly Ser His His His His His
       450                 455                 460
   cac                                                                 1395
   His
   465
<210> 22
   <211> 465
   <212> PRT
   <213> basf-yaad-Xa-rodA-his
<400> 22
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp Arg Ser Ile Glu Gly Arg Met Lys Phe Ser
       290                 295                 300
   Ile Ala Ala Ala Val Val Ala Phe Ala Ala Ser Val Ala Ala Leu Pro
   305                 310                 315                 320
   Pro Ala His Asp Ser Gln Phe Ala Gly Asn Gly Val Gly Asn Lys Gly
                   325                 330                 335
   Asn Ser Asn Val Lys Phe Pro Val Pro Glu Asn Val Thr Val Lys Gln
               340                 345                 350
   Ala Ser Asp Lys Cys Gly Asp Gln Ala Gln Leu Ser Cys Cys Asn Lys
           355                 360                 365
   Ala Thr Tyr Ala Gly Asp Thr Thr Thr Val Asp Glu Gly Leu Leu Ser
       370                 375                 380
   Gly Ala Leu Ser Gly Leu Ile Gly Ala Gly Ser Gly Ala Glu Gly Leu
   385                 390                 395                 400
   Gly Leu Phe Asp Gln Cys Ser Lys Leu Asp Val Ala Val Leu Ile Gly
                   405                 410                 415
   Ile Gln Asp Leu Val Asn Gln Lys Cys Lys Gln Asn Ile Ala Cys Cys
               420                 425                 430
   Gln Asn Ser Pro Ser Ser Ala Asp Gly Asn Leu Ile Gly Val Gly Leu
           435                 440                 445
   Pro Cys Val Ala Leu Gly Ser Ile Leu Gly Ser His His His His His
       450                 455                 460
   His
   465
<210> 23
   <211> 1407
   <212> DNA
   <213> basf-yaad-Xa-BASF1-his
<220>
   <221> CDS
   <222> (1)..(1407)
   <223>
<400> 23
   atg gct caa aca ggt act gaa cgt gta aaa cgc gga atg gca gaa atg        48
   Met Ala Gln Thr Gly Thr Glu Arg Val Lys Arg Gly Met Ala Glu Met
   1               5                   10                  15
   caa aaa ggc ggc gtc atc atg gac gtc atc aat gcg gaa caa gcg aaa        96
   Gln Lys Gly Gly Val Ile Met Asp Val Ile Asn Ala Glu Gln Ala Lys
               20                  25                  30
   atc gct gaa gaa gct gga gct gtc gct gta atg gcg cta gaa cgt gtg       144
   Ile Ala Glu Glu Ala Gly Ala Val Ala Val Met Ala Leu Glu Arg Val
           35                  40                  45
   cca gca gat att cgc gcg gct gga gga gtt gcc cgt atg gct gac cct       192
   Pro Ala Asp Ile Arg Ala Ala Gly Gly Val Ala Arg Met Ala Asp Pro
       50                  55                  60
   aca atc gtg gaa gaa gta atg aat gca gta tct atc ccg gta atg gca       240
   Thr Ile Val Glu Glu Val Met Asn Ala Val Ser Ile Pro Val Met Ala
   65                  70                  75                  80
   aaa gcg cgt atc gga cat att gtt gaa gcg cgt gtg ctt gaa gct atg       288
   Lys Ala Arg Ile Gly His Ile Val Glu Ala Arg Val Leu Glu Ala Met
                   85                  90                  95
   ggt gtt gac tat att gat gaa agt gaa gtt ctg acg ccg gct gac gaa       336
   Gly Val Asp Tyr Ile Asp Glu Ser Glu Val Leu Thr Pro Ala Asp Glu
               100                 105                 110
   gaa ttt cat tta aat aaa aat gaa tac aca gtt cct ttt gtc tgt ggc       384
   Glu Phe His Leu Asn Lys Asn Glu Tyr Thr Val Pro Phe Val Cys Gly
           115                 120                 125
   tgc cgt gat ctt ggt gaa gca aca cgc cgt att gcg gaa ggt gct tct       432
   Cys Arg Asp Leu Gly Glu Ala Thr Arg Arg Ile Ala Glu Gly Ala Ser
       130                 135                 140
   atg ctt cgc aca aaa ggt gag cct gga aca ggt aat att gtt gag gct       480
   Met Leu Arg Thr Lys Gly Glu Pro Gly Thr Gly Asn Ile Val Glu Ala
   145                 150                 155                 160
   gtt cgc cat atg cgt aaa gtt aac gct caa gtg cgc aaa gta gtt gcg       528
   Val Arg His Met Arg Lys Val Asn Ala Gln Val Arg Lys Val Val Ala
                   165                 170                 175
   atg agt gag gat gag cta atg aca gaa gcg aaa aac cta ggt gct cct       576
   Met Ser Glu Asp Glu Leu Met Thr Glu Ala Lys Asn Leu Gly Ala Pro
               180                 185                 190
   tac gag ctt ctt ctt caa att aaa aaa gac ggc aag ctt cct gtc gtt     624
   Tyr Glu Leu Leu Leu Gln Ile Lys Lys Asp Gly Lys Leu Pro Val Val
           195                 200                 205
   aac ttt gcc gct ggc ggc gta gca act cca gct gat gct gct ctc atg     672
   Asn Phe Ala Ala Gly Gly Val Ala Thr Pro Ala Asp Ala Ala Leu Met
       210                 215                 220
   atg cag ctt ggt gct gac gga gta ttt gtt ggt tct ggt att ttt aaa     720
   Met Gln Leu Gly Ala Asp Gly Val Phe Val Gly Ser Gly Ile Phe Lys
   225                 230                 235                 240
   tca gac aac cct gct aaa ttt gcg aaa gca att gtg gaa gca aca act     768
   Ser Asp Asn Pro Ala Lys Phe Ala Lys Ala Ile Val Glu Ala Thr Thr
                   245                 250                 255
   cac ttt act gat tac aaa tta atc gct gag ttg tca aaa gag ctt ggt     816
   His Phe Thr Asp Tyr Lys Leu Ile Ala Glu Leu Ser Lys Glu Leu Gly
               260                 265                 270
   act gca atg aaa ggg att gaa atc tca aac tta ctt cca gaa cag cgt     864
   Thr Ala Met Lys Gly Ile Glu Ile Ser Asn Leu Leu Pro Glu Gln Arg
           275                 280                 285
   atg caa gaa cgc ggc tgg aga tct att gaa ggc cgc atg aag ttc tcc     912
   Met Gln Glu Arg Gly Trp Arg Ser Ile Glu Gly Arg Met Lys Phe Ser
       290                 295                 300
   gtc tcc gcc gcc gtc ctc gcc ttc gcc gcc tcc gtc gcc gcc ctc cct     960
   Val Ser Ala Ala Val Leu Ala Phe Ala Ala Ser Val Ala Ala Leu Pro
   305                 310                 315                 320
   cag cac gac tcc gcc gcc ggc aac ggc aac ggc gtc ggc aac aag ttc    1008
   Gln His Asp Ser Ala Ala Gly Asn Gly Asn Gly Val Gly Asn Lys Phe
                   325                 330                 335
   cct gtc cct gac gac gtc acc gtc aag cag gcc acc gac aag tgc ggc    1056
   Pro Val Pro Asp Asp Val Thr Val Lys Gln Ala Thr Asp Lys Cys Gly
               340                 345                 350
   gac cag gcc cag ctc tcc tgc tgc aac aag gcc acc tac gcc ggc gac    1104
   Asp Gln Ala Gln Leu Ser Cys Cys Asn Lys Ala Thr Tyr Ala Gly Asp
           355                 360                 365
   gtc ctc acc gac atc gac gag ggc atc ctc gcc ggc ctc ctc aag aac    1152
   Val Leu Thr Asp Ile Asp Glu Gly Ile Leu Ala Gly Leu Leu Lys Asn
       370                 375                 380
   ctc atc ggc ggc ggc tcc ggc tcc gag ggc ctc ggc ctc ttc gac cag    1200
   Leu Ile Gly Gly Gly Ser Gly Ser Glu Gly Leu Gly Leu Phe Asp Gln
   385                 390                 395                 400
   tgc gtc aag ctc gac ctc cag atc tcc gtc atc ggc atc cct atc cag    1248
   Cys Val Lys Leu Asp Leu Gln Ile Ser Val Ile Gly Ile Pro Ile Gln
                   405                 410                 415
   gac ctc ctc aac cag gtc aac aag cag tgc aag cag aac atc gcc tgc    1296
   Asp Leu Leu Asn Gln Val Asn Lys Gln Cys Lys Gln Asn Ile Ala Cys
               420                 425                 430
   tgc cag aac tcc cct tcc gac gcc acc ggc tcc ctc gtc aac ctc ggc    1344
   Cys Gln Asn Ser Pro Ser Asp Ala Thr Gly Ser Leu Val Asn Leu Gly
           435                 440                 445
   ctc ggc aac cct tgc atc cct gtc tcc ctc ctc cat atg gga tct cat    1392
   Leu Gly Asn Pro Cys Ile Pro Val Ser Leu Leu His Met Gly Ser His
       450                 455                 460
   cac cat cac cat cac                                                  1407
   His His His His His
   465
<210> 24
   <211> 469
   <212> PRT
   <213> basf-yaad-Xa-BASF1-his
<400> 24
   Met Ala Gln Thr Gly Thr Glu Arg Val Lys Arg Gly Met Ala Glu Met
   1               5                   10                  15
   Gln Lys Gly Gly Val Ile Met Asp Val Ile Asn Ala Glu Gln Ala Lys
               20                  25                  30
   Ile Ala Glu Glu Ala Gly Ala Val Ala Val Met Ala Leu Glu Arg Val
           35                  40                  45
   Pro Ala Asp Ile Arg Ala Ala Gly Gly Val Ala Arg Met Ala Asp Pro
       50                  55                  60
   Thr Ile Val Glu Glu Val Met Asn Ala Val Ser Ile Pro Val Met Ala
   65                  70                  75                  80
   Lys Ala Arg Ile Gly His Ile Val Glu Ala Arg Val Leu Glu Ala Met
                   85                  90                  95
   Gly Val Asp Tyr Ile Asp Glu Ser Glu Val Leu Thr Pro Ala Asp Glu
               100                 105                 110
   Glu Phe His Leu Asn Lys Asn Glu Tyr Thr Val Pro Phe Val Cys Gly
           115                 120                 125
   Cys Arg Asp Leu Gly Glu Ala Thr Arg Arg Ile Ala Glu Gly Ala Ser
       130                 135                 140
   Met Leu Arg Thr Lys Gly Glu Pro Gly Thr Gly Asn Ile Val Glu Ala
   145                 150                 155                 160
   Val Arg His Met Arg Lys Val Asn Ala Gln Val Arg Lys Val Val Ala
                   165                 170                 175
   Met Ser Glu Asp Glu Leu Met Thr Glu Ala Lys Asn Leu Gly Ala Pro
               180                 185                 190
   Tyr Glu Leu Leu Leu Gln Ile Lys Lys Asp Gly Lys Leu Pro Val Val
           195                 200                 205
   Asn Phe Ala Ala Gly Gly Val Ala Thr Pro Ala Asp Ala Ala Leu Met
       210                 215                 220
   Met Gln Leu Gly Ala Asp Gly Val Phe Val Gly Ser Gly Ile Phe Lys
   225                 230                 235                 240
   Ser Asp Asn Pro Ala Lys Phe Ala Lys Ala Ile Val Glu Ala Thr Thr
                   245                 250                 255
   His Phe Thr Asp Tyr Lys Leu Ile Ala Glu Leu Ser Lys Glu Leu Gly
               260                 265                 270
   Thr Ala Met Lys Gly Ile Glu Ile Ser Asn Leu Leu Pro Glu Gln Arg
           275                 280                 285
   Met Gln Glu Arg Gly Trp Arg Ser Ile Glu Gly Arg Met Lys Phe Ser
       290                 295                 300
   Val Ser Ala Ala Val Leu Ala Phe Ala Ala Ser Val Ala Ala Leu Pro
   305                 310                 315                 320
   Gln His Asp Ser Ala Ala Gly Asn Gly Asn Gly Val Gly Asn Lys Phe
                   325                 330                 335
   Pro Val Pro Asp Asp Val Thr Val Lys Gln Ala Thr Asp Lys Cys Gly
               340                 345                 350
   Asp Gln Ala Gln Leu Ser Cys Cys Asn Lys Ala Thr Tyr Ala Gly Asp
           355                 360                 365
   Val Leu Thr Asp Ile Asp Glu Gly Ile Leu Ala Gly Leu Leu Lys Asn
       370                 375                 380
   Leu Ile Gly Gly Gly Ser Gly Ser Glu Gly Leu Gly Leu Phe Asp Gln
   385                 390                 395                 400
   Cys Val Lys Leu Asp Leu Gln Ile Ser Val Ile Gly Ile Pro Ile Gln
                   405                 410                 415
   Asp Leu Leu Asn Gln Val Asn Lys Gln Cys Lys Gln Asn Ile Ala Cys
               420                 425                 430
   Cys Gln Asn Ser Pro Ser Asp Ala Thr Gly Ser Leu Val Asn Leu Gly
           435                 440                 445
   Leu Gly Asn Pro Cys Ile Pro Val Ser Leu Leu His Met Gly Ser His
       450                 455                 460
   His His His His His
   465

## Beschreibung

### Stand der Technik

Hydrophobine sind kleine Proteine von etwa 100 AS, die charakteristisch für filamentöse Pilze sind und nicht in anderen Organismen vorkommen. Kürzlich wurden Hydrophobin-ähnliche Proteine in *Streptomyces coelicolorentdeckt,* die als "Chapline" bezeichnet werden und ebenfalls hoch oberflächenaktive Eigenschaften haben. An Wasser/Luft-Grenzflächen können sich Chapline zu Amyloid-ähnlichen Fibrillen assemblieren (Classen et al. 2003 Genes Dev 1714-1726 ; Elliot et al. 2003, Genes Dev. 17, 1727-1740).

Hydrophobine sind in einer wasserunlöslichen Form auf der Oberfläche von verschiedenen pilzlichen Strukturen, wie z.B. Lufthyphen, Sporen, Fruchtkörpern, verteilt. Die Gene für Hydrophobine konnten aus Ascomyzeten, Deuteromyzeten und Basidiomyzeten isoliert werden. Einige Pilze enthalten mehr als ein Hydrophobingen, z.B. *Schizophyllum commune, Coprinus cinereus, Aspergillus nidulans.* Offensichtlich sind verschiedene Hydrophobine in unterschiedlichen Stadien der pilzlichen Entwicklung involviert. Die Hydrophobine sind dabei vermutlich verantwortlich für unterschiedliche Funktionen (van Wetter et al., 2000, Mol. Microbiol., 36, 201-210; Kershaw et al. 1998, Fungal Genet. Biol, 1998, 23, 18-33).

Als biologische Funktion für Hydrophobine wird neben der Reduzierung der Oberflächenspannung von Wasser zur Generierung von Lufthyphen auch die Hydrophobisierung von Sporen beschrieben (Wösten et al. 1999, Curr. Biol., 19, 1985-88; Bell et al. 1992, Genes Dev., 6, 2382-2394). Weiterhin dienen Hydrophobine zur Auskleidung von Gaskanälen in Fruchtkörpern von Flechten und als Komponenten im Erkennungssystem von pflanzlichen Oberflächen durch pilzliche Pathogene (Lugones et al. 1999, Mycol. Res., 103, 635-640; Hamer & Talbot 1998, Curr. Opinion Microbiol., Band 1, 693-697).

Komplementationsexperimente haben gezeigt, dass Hydrophobine sich innerhalb einer Klasse bis zu einem gewissen Grad funktionell ersetzen können. Hydrophobine umfassende Fusionsproteine sind z.B. aus WO00/58342 bekannt. Die bisher bekannten Hydrophobine lassen sich mit üblichen proteinchemischen Reinigungs- und Isolationsverfahren nur in mässiger Ausbeute und Reinheit darstellen. Auch Versuche, mit Hilfe von gentechnischen Verfahren, grössere Mengen an Hydrophobinen bereitzustellen, waren bisher nicht erfolgreich.

### Aufgabenstellung

Es bestand die Aufgabe, neuartige Hydrophobine und deren Herstellungsverfahren bereitzustellen, die eine wirtschaftliche Produktion der Hydrophobine und deren Einsatz auf verschiedenen technischen Gebieten erlauben.

### Beschreibung der Erfindung

Gegenstand der Erfindung sind Polypeptide der allgemeinen Strukturformel (I)

Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)

wobei X für jede der 20 natürlich vorkommenden Aminosäuren steht,
n und m für Zahlen zwischen 0 und 500 stehen,
C für Cystein steht,
mit der Maßgabe, dass wenigstens eine der mit Xₙ oder Xₘ abgekürzten Peptidsequenzen für eine mindestens 20 Aminosäuren lange Peptidsequenz steht, die natürlicherweise nicht mit einem Hydrophobin verknüpft ist, dadurch gekennzeichnet, dass die Struktkurformel (I)
   (i) nur ein Hydrophobin umfasst,
   (ii) eine Sequenz SEQ ID NO:2 umfasst und
   (iii) daß Xₙ oder Xₘ eine Polypeptidsequenz ausgewählt aus SEQ ID NO: 16 oder Fragmenten von SEQ ID NO:16 mit 80-99% der Sequenz umfasst,
die nach Beschichten einer Glasoberfläche eine Kontaktwinkeländerung von mindestens 20° bewirken.

Die mit C¹ bis C⁸ benannten Cysteine können in den erfindungsgemässen Proteinen entweder reduziert vorliegen oder miteinander Disulfidbrücken ausbilden. Besonders bevorzugt ist die intramolekulare Ausbildung von C-C Brücken, insbesondere die mit mindestens einer, bevorzugt 2, besonders bevorzugt 3 und ganz besonder bevorzugt 4 intramolekularen Disulfidbrücken ausgewählt aus der folgenden Gruppe: C¹ mit C²; C³ mit C⁴, C⁵ mit C⁶, C⁷ mit C⁸.

Falls in den mit X bezeichneten Positionen auch Cysteine verwendet werden, kann sich die Nummerierung der einzelnen Cysteinpositionen in den allgemeinen Formeln entsprechend verändern.

Besonders geeignete Fusionspartner sind Polypeptide, die natürlicherweise in Mikroorganismen, insbesondere in E. coli oder Bacillus subtilis vorkommen. Beispiele für solche Fusionspartner sind die Sequenzen yaad (SEQ ID NO:15 und 16). Gut geeignet sind auch Fragmente dieser genannten Sequenzen, die nur 80-98% der genannten Sequenzen umfassen, oder bei denen einzelne Aminosäuren, bzw. Nukleotide gegenüber der genannten Sequenz verändert sind. Beispielsweise können am C-terminalen Ende der Sequenzen yaad noch zusätzliche Aminosäuren, insbesondere zwei zusätzliche Aminosäuren, bevorzugt die Aminosäuren Arg, Ser, angefügt sein.

Weiterhin können auch an den Verknüpfungsstellen zweier Fusionsparter zusätzliche Aminosäuren eingefügt werden, die sich dadurch ergeben, dass auf der Nukleinsäureebene Erkennungsstellen für Restriktionsendonukleasen entweder neu kreiert oder inaktiviert werden.

Die erfindungsgemässen Proteine können auch noch in ihrer Polypeptidsequenz modifiziert sein, beispielsweise durch Glycosilierung, Acetylierung oder auch durch chemische Quervernetzung beispielsweise mit Glutardialdehyd.

Bevorzugte Ausführungsformen der beschriebenen Erfindung sind Polypeptide mit der allgemeinen Strukturformel (I) (II) oder (III), wobei diese Strukturformel mindestens ein Hydrophobin der Klasse I, bevorzugt mindestens ein Hydrophobin dewA, rodA, hypA, hypB, sc3, basf1, basf2, oder Teile oder Derivate davon umfasst. Die genannten Hydrophobine sind im nachfolgenden Sequenzprotokoll strukturell charakterisiert. Es können auch mehrere Hydrophobine, bevorzugt 2 oder 3, gleicher oder unterschiedlicher Struktur miteinander verknüpft und mit einer entsprechenden geeigneten Polypeptidsequenz, die natürlicherweise nicht mit einem Hydrophobin verbunden ist, verknüpft werden.

Besonders bevorzugte Ausführungsformen der vorliegenden Erfindung sind die neuen Proteine mit der in SEQ ID NO: 20, 22, 24 dargestellen Polypeptidsequenzen sowie den dafür codierenden Nukleinsäuresequenzen, insbesondere den Sequenzen gemäss SEQ ID NO: 19, 21, 23. Auch Proteine, die sich ausgehend von den in SEQ ID NO. 22, 22 oder 24 dargestellten Polypeptidsequenzen durch Austausch, Insertion oder Deletion von mindestens einer, bis hin zu 10. bevorzugt 5 , besonders bevorzugt 5% aller Aminosäuren ergeben, und die die biologische Eigenschaft der Ausgangsproteine noch zu mindestens 50% besitzen, sind besonders bevorzugte Ausführungsformen. Unter biologischer Eigenschaft der Proteine wird hierbei die Änderung des Kontaktwinkels wie in Beispiel 10 beschrieben, verstanden.

Die erfindungsgemässen Proteine tragen an mindestens einer Position, die mit Xₙ oder Xₘ abgekürzt wird, eine Polypeptidsequenz aus mindestens 20 bevorzugt mindestens 35 besonders bevorzugt mindestens 50 und insbesonders mindestens 100 Aminosäuren (im folgenden auch Fusionspartner genannt), die nicht natürlicherweise mit einem Hydrophobin verknüpft ist. Damit soll ausgedrückt werden, dass die erfindungsgemässen Proteine aus einem Hydrophobinteil und einem Fusionspartnerteil bestehen, die in der Natur nicht zusammen in dieser Form vorkommen.

Der Fusionspartnerteil kann aus einer Vielzahl von Proteinen ausgewählt werden. Es können auch mehrere Fusionspartner mit einem Hydrophobinteil verknüpft werden, beispielsweise am Aminoterminus (Xₙ) und am Carboxyterminus (Xₘ) des Hydrophobinteils. Es können aber auch beispielsweise zwei Fusionspartner mit einer Position (Xₙ oder Xₘ) des erfindungsgemässen Proteins verknüpft werden.

Als Fusionspartner sind besonders bevorzugt solche Polypeptidsequenzen, die dazu führen, dass das erfindungsgemässe Protein zur Beschichtung von Glasoberflächen fähig ist und die mit Protein behandelte Glasoberfläche resistent wird gegenüber einer Detergenzbehandlung, wie sie im experimentellen Teil (Beispiel 10) ausführlich beschrieben ist (z.B. 1% SDS / 80°C / 10 min) ist.

Besonders geeignete Fusionspartner sind Polypeptide, die natürlicherweise in Mikroorganismen, insbesondere in E. coli oder Bacillus subtilis vorkommen. Beispiele für solche Fusionspartner sind die Sequenzen yaad (SEQ ID NO:15 und 16), yaae(SEQ ID NO:17 und 18), und Thioredoxin. Gut geeignet sind auch Fragmente oder Derivate dieser genannten Sequenzen, die nur einen Teil, bevorzugt 70-99%, besonders bevorzugt 80-98% der genannten Sequenzen umfassen, oder bei denen einzelne Aminosäuren, bzw. Nukleotide gegenüber der genannten Sequenz verändert sind. Beispielsweise können am C-terminalen Ende der Sequenzen yaad und yaae noch zusätzliche Aminosäuren, insbesondere zwei zusätzliche Aminosäuren, bevorzugt die Aminosäuren Arg, Ser, angefügt sein. Ferner können bevorzugt in der Sequenz yaae zusätzliche Aminosäuren gegenüber der natürlich vorkommenden Sequenz eingefügt sein, beispielsweise die Aminosäure Nr. 2 (Gly) in SEQ ID NO: 17 und 18.

Weiterhin können auch an den Verknüpfungsstellen zweier Fusionsparter zusätzliche Aminosäuren eingefügt werden, die sich dadurch ergeben, dass auf der Nukleinsäureebene Erkennungsstellen für Restriktionsendonukleasen entweder neu kreiert oder inaktiviert werden.

Die erfindungsgemässen Proteine können auch noch in ihrer Polypeptidsequenz modifiziert sein, beispielsweise durch Glycosilierung, Acetylierung oder auch durch chemische Quervernetzung beispielsweise mit Glutardialdehyd.

Eine Eigenschaft der erfindungsgemässen Proteine ist die Änderung von Oberflächeneigenschaften, wenn die Oberflächen mit den Proteinen beschichtet werden. Die Änderung der Oberflächeneigenschaften lässt sich experimentell dadurch bestimmen, dass der Kontaktwinkel eines Wassertropfens vor und nach der Beschichtung der Oberfläche mit dem erfindungsgemässen Protein gemessen wird und die Differenz der beiden Messungen ermittelt wird.

Die genauen experimentellen Bedingungen zur Messung des Kontaktwinkels sind im experimentellen Teil in Beispiel 10 niedergelegt. Unter diesen Bedingungen besitzen die erfindungsgemässen Proteine die Eigenschaft, den Kontaktwinkel um mindestens 20, bevorzugt 25, besonders bevorzugt 30 Grad zu vergrössern.

Im Hydrophobinteil der bisher bekannten Hydrophobine sind die Positionen der polaren und unpolaren Aminosäuren konserviert, was sich in einem charakteristischen Hydrophobizitätsplot äußert. Unterschiede in den biophysikalischen Eigenschaften und in der Hydrophobizität führten zur Einteilung der bisher bekannten Hydrophobine in zwei Klassen, I und II (Wessels et al. 1994, Ann. Rev. Phytopathol., 32, 413-437).

Die assemblierten Membranen aus Klasse I Hydrophobinen sind hochgradig unlöslich (selbst gegenüber 1% SDS bei erhöhter Temperatur) und können nur durch konzentrierte Trifluoressigsäure (TFA), bzw. Ameisensäure wieder dissoziiert werden. Im Gegensatz dazu sind die assemblierten Formen von Klasse II Hydrophobinen weniger stabil. Sie können bereits durch 60%iges Ethanol, bzw. 1 % SDS (bei Raumtemperatur) wieder aufgelöst werden.

Ein Vergleich der Aminosäuresequenzen zeigt, dass die Länge des Bereichs zwischen Cystein C³ und C⁴ bei Klasse II Hydrophobinen deutlich kürzer ist, als bei Hydrophobinen der Klasse I.

Klasse II Hydrophobine weisen weiterhin mehr geladene Aminosäuren als Klasse I auf.

Ein weiterer Gegenstand der Erfindung sind Verfahren zur Herstellung der erfindungsgemässen Proteine. Diese Polypeptide lassen sich chemisch durch bekannte Verfahren der Peptidsynthese beispielsweise durch Festphasensynthese nach Merrifield herstellen.

Besonders geeignet sind jedoch gentechnische Verfahren, bei denen eine für den Fusionspartner und eine für den Hydrophobinteil codierende Nukleinsäuresequenz, insbesondere DNA-Sequenz, so kombiniert werden, dass in einem Wirtsorganismus durch Genexpression der kombinierten Nukleinsäuresequenz das gewünschte Protein erzeugt wird.

Geeignete Wirtsorganismen (Produktionsorganismen) können dabei Prokaryonten (einschliesslich der Archaea) oder Eukaryonten sein, besonders Bakterien einschliesslich Halobacterien und Methanococcen, Pilze, Insektenzellen, Pflanzenzellen und Säugerzellen, besonders bevorzugt Escherichia coli, Bacillus subtilis, Bacillus. megaterium, Aspergillus oryzea, Aspergillus nidulans, Aspergillus niger, Pichia pastoris, Pseudomonas spec., Lactobacillen, Hansenula polymorpha, Trichoderma reesei, SF9 (bzw. verwandte Zellen) u.a.

Gegenstand der Erfindung sind ausserdem Expressionskonstrukte, enthaltend unter der genetischen Kontrolle regulativer Nukleinsäuresequenzen eine für ein erfindungsgemässes Polypeptid kodierende Nukleinsäuresequenz, sowie Vektoren, umfassend wenigstens eines dieser Expressionskonstrukte.

Vorzugsweise umfassen solche erfindungsgemässen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer"operativen Verknüpfung"versteht man die sequentielle Anordnung von Promotor, kodierender Sequenz, Terminator und gegebenenfalls weitererregulativer Elemente derart, dass jedes der regulativen Elemente seine Funktion bei der Expression der kodierenden Sequenz bestimmungsgemäss erfüllen kann.

Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z. B. beschrieben in Goeddel, Gene Expression Techno- logy : Methods in Enzymology 185, Academic Press, San Diego, CA (1990).

Zusätzlich zu diesenRegulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde.

Ein bevorzugtesNukleinsäurekonstrukt enthält vorteilhafterweise auch eine oder mehrere der schon erwähnten"Enhancer"Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren.

Die erfindungsgemässen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemässe Verfahren sind beispielsweise in Promotoren wie cos-, tac-, trp-, tet-, trp-tet-, lpp-, lac-,lpp-lac-,laclq-T7-, T5-, T3-, gal-, trc-, ara-,rhaP(rhaPBAD) SP6-, lambda-PR-oder imlambda-P-Promotor enthalten, die vorteilhafterweise in gram-negativen Bakterien Anwendung finden. Weitere vorteilhafte Regulationssequenzen sind beispielsweise in den gram-positiven Promotoren amy und SP02, in den Hefe-oder Pilzpromotoren ADC1,MFalpha, AC, P-60, CYC1, GAPDH, TEF, rp28, ADH enthalten.

Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind ausser Plasmiden und Phagen auch alle anderen dem Fachmann bekannten Vektoren, also z. B. Viren, wie SV40, CMV, Baculovirus und Adenovirus, Transposons, IS- Elemente, Phasmide, Cosmide, und lineare oder zirkuläre DNA, sowie das Agrobacterium-System zu verstehen.

Diese Vektoren können autonom im Wirtsorganismus repliziert oder chromosomal repliziert werden. Diese Vektoren stellen eine weitere Ausgestaltung der Erfindung dar. Geeig- nete Plasmide sind beispielsweise in E. coli pLG338,pACYC184, pBR322, pUC18,pUC19, pKC30, pRep4, pHS1, pKK223-3, pDHE19.2, pHS2, pPLc236, pMBL24, pLG200, pUR290,pIN-III"3-B1, tgt11 oderpBdCI, in StreptomycesplJ101, pIJ364,pIJ702 oderplJ361, in Bacillus pUB110, pC194 oder pBD214, in Corynebacterium pSA77 oder pAJ667, in Pilzen pALS1, pIL2 oder pBB116, in Hefen 2alpha, pAG-1, YEp6, YEp13 oder pEMBLYe23 oder in Pflanzen pLGV23,pGHIac+,pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden. Vorteilhafterweise enthält das Nukleinsäurekonstrukt zur Expression der weiteren enthaltenen Gene zusätzlich noch 3'-und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotorenund/oder"Enhancer"verwendet werden. Daneben ist aber auch eine Verstärkung der Translation möglich, indem beispielsweise die Stabilität dermRNA verbessert wird.

In einer weiteren Ausgestaltungsform des Vektors kann der das erfindungsgemässe-Nukleinsäurekonstrukt oder die erfindungsgemässe Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemässen Nukleinsäure bestehen.

Für eine optimale Expression heterologer Gene in Organismen ist es vorteilhaft die Nukleinsäuresequenzen entsprechend des im Organismus verwendeten spezifischen "codon usage"zu verändern. Der"codon usage"lässt sich anhand von Computerauswertungen anderer, bekannter Gene des betreffenden Organismus leicht ermitteln.

Die Herstellung einer erfindungsgemässen Expressionskassette erfolgt durch Fusion eines geeigneten Promotors mit einer geeigneten kodierenden Nukleotidsequenz sowie einem Terminator-oder Polyadenylierungssignal. Dazu verwendet man gängige Rekombinations-und Klonierungstechniken, wie sie beispielsweise in T. Maniatis, E. F.Fritsch und J. Sambrook, Molecular Cloning : A Laboratory Manual, Cold Spring Harbor Laboratory, Cold Spring Harbor, NY (1989) sowie in T. J. Silhavy, M. L. Berman und L. W. Enquist, Experiments with Gene Fusions, Cold Spring Harbor Laboratory, Cold Spring Harbor, NY (1984) und in Ausubel, F. M. etal., Current Protocols in Molecular Biology, Greene Publishing Assoc. andWiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus"Cloning Vectors" (Pouwels P. H. etal., Hrsg, Elsevier, Amsterdam-New York-Oxford, 1985) entnommen werden.

Mit Hilfe der erfindungsgemässen Vektoren sind rekombinante Mikroorganismen herstellbar, welche beispielsweise mit wenigstens einem erfindungsgemässen Vektor transformiert sind und zur Produktion der erfindungsgemässen Polypeptide eingesetzt werden können. Vorteilhafterweise werden die oben beschriebenen erfindungsgemässen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F.Ausubel etal., Hrsg., Wiley Interscience, New York 1997, oder Sambrook et al. Molecular Cloning : A Laboratory Manual. 2. Aufl., Cold Spring Harbor Laboratory, Cold Spring Harbor Laboratory Press, Cold Spring Harbor, NY, 1989 beschrieben.

Erfindungsgemäss sind auch homolog rekombinierte Mikroorganismen herstellbar. Dazu wird ein Vektor hergestellt, der zumindest einen Abschnitt eines erfindungsgemässen Gens oder einer kodierenden Sequenz enthält, worin gegebenenfalls wenigstens eine Aminosäure-Deletion,-Addition oder-Substitution eingebracht worden ist, um die erfin- dungsgemässe Sequenz zu verändern, z. B. funktionell zu disruptieren ("Knock-out"- Vektor). Die eingebrachte Sequenz kann z. B. auch ein Homologes aus einem verwandten Mikroorganismus sein oder aus einer Säugetier-, Hefe-oder Insektenquelle abgeleitet sein. Der zur homologen Rekombination verwendete Vektor kann alternativ derart ausgestaltet sein, dass das endogene Gen bei homologer Rekombina- tion mutiert oder anderweitig verändert ist, jedoch noch das funktionelle Protein kodiert (z. B. kann der stromaufwärts gelegene regulatorische Bereich derart verändert sein, dass dadurch die Expression des endogenen Proteins verändert wird). Der veränderte Abschnitt des erfindungsgemässen Gens ist im homologen Rekombinationsvektor. Die Konstruktion geeigneter Vektoren zur homologen Rekombination ist z. B. beschrieben in Thomas, K. R. und Capecchi, M. R. (1987) Cell 51 : 503.

Als rekombinante Wirtsorganismen für die erfindungsgemässe Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet.

Die im erfindungsgemässen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen0 C und 100 C, bevorzugt zwischen 10 C bis60 C unter Sauerstoffbegasung angezogen. Dabei kann der pH der Nährflüssigkeit auf einen festen Wert gehalten werden, das heisst während der Anzucht reguliert werden oder nicht. Die Anzucht kann"batch"-weise,"semi batch"-weise oder kontinuierlich erfolgen. Nährstoffe können zu beginn der Fermentation vorgelegt oder semikontinuierlich oder kontinuierlich nachgefüttert werden. Die Enzyme können nach dem in den Beispielen beschriebe- nen Verfahren aus den Organismen isoliert werden oder als Rohextrakt für die Reakti- on verwendet werden.

Gegenstand der Erfindung sind weiterhin Verfahren zur rekombinanten Herstellung erfindungsgemässe Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in grosstechnischem Massstab produziert werden, falls dies erwünscht ist. Der rekombinante Mikroorganismus kann nach bekannten Verfahren kultiviert und fermentiert werden. Bakterien können beispielsweise in TB-oder LB-Medium und bei einer Temperatur von 20 bis40°C und einem pH-Wert von 6 bis 9 vermehrt werden. Im Einzelnen werden geeignete Kultivierungsbedingungen beispielsweise in T. Maniatis, E. F. Fritsch and J. Sambrook, Molecular Cloning : A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien,lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q- Sepharose-Chromatographie, lonenaustausch-Chromatographie und hydrophobe Chromatographie, sowie mit anderen üblichen Verfahren wie Ultrafiltration,Kristallisati- on, Aussalzen, Dialyse und nativerGelelektrophorese. Geeignete Verfahren werden beispielsweise in Cooper, F. G., Biochemische Arbeitsmethoden, Verlag Water de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z. B. einer einfacheren Reinigung dienen. Derartige geeignete Modifikationen sind beispielsweise als Anker fungierende sogenannte"Tags", wie z. B. die als Hexa-Histidin-Anker bekannte Modifikation oder Epitope, die als Antigene von Antikörpern erkannt werden können (beschrieben zum Beispiel inHarlow, E. and Lane, D., 1988, Antibodies : A Laboratory Manual. Cold Spring Harbor (N. Y.) Press). Weitere geeignete Tags sind z.B. HA, Calmodulin-BD, GST, MBD; Chitin-BD, Steptavidin-BD-Avi-Tag, Flag-Tag, T7 etc. Diese Anker können zur Anheftung der Proteine an einen festen Träger, wie z. B. einer Polymermatrix, dienen, die beispielsweise in einer Chromatographiesäule eingefüllt sein kann, oder an einer Mikrotiterplatte oder an einem sonstigen Träger verwendet werden kann. Die entsprechenden Reinigungsprotokolle sind von den kommerziellen Affinitäts-Tag-Anbietern erhältlich.

Viele Hydrophobin-beschichtete pilzliche Oberflächen (Sporen, Fruchtkörper, Myzel) zeigen mikroskopisch nachweisbare, charakteristische Strukturen, sogenannte Rodlets. Ähnliche Rodlets mit einer Stärke von ca. 10 nm können auch auf mit Hydrophobin beschichteten hydrophilen Oberflächen (z.B. Glas, Glimmer etc.) nachgewiesen werden (Wösten et al., 1993, Plant Cell, 5, 1567-1574).

Aufgrund der außergewöhnlichen Eigenschaften von Hydrophobinen zur Beschichtung von Oberflächen (z.B. widerstandsfähig gegenüber Detergentien wie z.B. 1 %iger SDS Lösung) besitzen diese Proteine ein hohes Potential für zahlreiche technische Anwendungen. In verschiedenen Patentschriften sind Beispiele für derartige Anwendungen benannt, auf die hiermit hinsichtlich der Anwendung von Hydrophobinen Bezug genommen wird.

| *Nr.* | *Prio* | *Anmelder* |
|---|---|---|
| WO 03/10331 | 23.07.2001 | Applied Nanosystems B.V. |
| WO 04/00880 | 21.06.2002 | Applied Nanosystems B.V. |
| WO 03/84508 | 04.04.2002 | Applied Nanosystems B.V. |
| WO 00/40968 | 05.01.1999 | Unilever N.V. (Hindustan Lever Ltd.) |
| EP-B 1 252 516 | 04.02.2000 | Applied Nanosystems B.V. Stichting voor de Technische Wetenschappen |
| EP-B 1257 571 | 04.02.2000 | Applied Nanosystems B.V. |
| WO 01/57066 | 04.02.2000 | Applied Nanosystems B.V. |
| WO 03/10331 | 23.07.2001 | Applied Nanosystems B.V. |
| WO 03/53383 | 14.12.2001 | L'Oreal |

Die technische Nutzung von Hydrophobinen, insbesondere solchen der Klasse I scheiterte bislang an einer effizienten Herstellungs- und Reinigungsmethode. Die bisher beschriebenen Verfahren, ausgehend von natürlichen Quellen (Sporen, Pilzmyzel etc.,) führen nur zu Materialmengen im mg-Maßstab. (z.B. WO 96/41882).

Ebenso erwiesen sich Ansätze über eine rekombinante Herstellung in verschiedenen Produktionsorganismen als äußerst aufwendig und wenig zufriedenstellend.

Die erfindungsgemässen Hydrophobin-Proteine besitzen sowohl in ihrer fusionierten Form, d.h. zusammen mit dem Fusionspartnerteil, als auch in isolierter Form die wünschenswerten Eigenschaften von Hydrophobinen. Man kann also die erfindungsgemässen Proteine sowohl direkt als Fusionsproteine als auch nach Abspaltung und Abtrennung des Fusionspartners als "reine" Hydrophobine verwenden.

Wenn eine Abtrennung des Fusionspartners vorgesehen ist, empfiehlt es sich eine potentielle Spaltstelle (spezifische Erkennungsstelle für Proteasen) in das Fusionsprotein zwischen Hydrophobinteil und Fusionspartnerteil einzubauen. Als Spaltstelle geeignet sind insbesondere solche Peptidsequenzen geeignet, die ansonsten weder im Hydrophobinteil noch im Fusionspartnerteil vorkommen, was sich mit bioinformatischen Tools leicht ermitteln lässt. Besonders geeignet sind beispielsweise BrCN-Spaltung an Methionin, oder durch Protease vermittelte Spaltlung mit Faktor Xa-, Enterokinase-, Thrombin, TEV-Spaltung (Tobacca etch virus Protease).

### Experimenteller Teil

### Beispiel 1

### Vorarbeiten für die Klonierung von yaad-His₆/ yaaE-His₆

Mit Hilfe der Oligonukleotide Hal570 und Hal571 (Hal 572/ Hal 573) wurde eine Polymerase Kettenreaktion durchgeführt. Als Template DNA wurde genomische DNA des Bakteriums Bacillus subtilis verwendet. Das erhaltene PCR Fragment enthielt die codierende Sequenz des Gens yaaD / yaaE aus Bacillus subtilis, und an den Enden je eine Ncol bzw. BgIII Restriktionsschnittstelle. Das PCR Fragment wurde gereinigt und mit den Restriktionsendonukleasen Ncol und BgIII geschnitten. Dieses DNA Fragment wurde als Insert verwendet, und in den zuvor mit den Restriktionsendonukleasen Ncol und Bglll linearisierten Vektor pQE60 der Firma Qiagen kioniert. Die so enstandenen Vektoren pQE60YAAD#2 / pQE60YaaE#5 können zur Expression von Proteinen bestehend aus, YAAD::HIS₆ bzw. YAAE::HIS₆ verwendet werden.
Hal570: gcgcgcccatggctcaaacaggtactga
Hal571: gcagatctccagccgcgttcttgcatac
Hal572: ggccatgggattaacaataggtgtactagg
Hal573: gcagatcttacaagtgccttttgcttatattcc

### Beispiel 2

### Klonierung von yaad-Hydrophobin DewA-His₆

Mit Hilfe der Oligonukleotide KaM 416 und KaM 417 wurde eine Polymerase Kettenreaktion durchgeführt. Als Template DNA wurde genomische DNA des Schimmelpilzes Aspergillus nidulans verwendet. Das erhaltene PCR Fragment enthielt die codierende Sequenz des Hydrophobin Gens dewA und einer N-Terminalen FaktorXa Proteinase Schnittstelle. Das PCR Fragment wurde gereinigt und mit der Restriktionsendonuklease BamHI geschnitten. Dieses DNA Fragment wurde als Insert verwendet, und in den zuvor mit der Restriktionsendonuklease BgIII linearisierten Vektor pQE60YAAD#2 kloniert.

Der so enstandene Vektor #508 kann zur Expressions eines Fusionsproteins bestehend aus, YAAD::Xa::dewA::HIS₆ verwendet werden.
KaM416: GCAGCCCATCAGGGATCCCTCAGCCTTGGTACCAGCGC
KaM417: CCCGTAGCTAGTGGATCCATTGAAGGCCGCAT-GAAGTTCTCCGTCTCCGC

### Beispiel 3

### Klonierung von yaad-Hydrophobin RodA-His₆

Die Klonierung des Plasmids #513 erfolgte analog zu Plasmid #508 unter Verwendung der Oligonukleotide KaM 434 und KaM 435.
KaM434: GCTAAGCGGATCCATTGAAGGCCGCATGAAGTTCTCCATTGCTGC
KaM435: CCAATGGGGATCCGAGGATGGAGCCAAGGG

### Beispiel 4

### Klonierung von yaad-Hydrophobin BASF1-His₆

Die Klonierung des Plasmids #507 erfolgte analog zu Plasmid #508 unter Verwendung der Oligonukleotide KaM 417 und KaM 418.

Als Template DNA wurde ein künstlich synthetisierte DNA Sequenz - Hydrophobin BASF1 -eingesetzt (siehe Anhang).
KaM417:CCCGTAGCTAGTGGATCCATTGAAGGCCGCAT-GAAGTTCTCCGTCTCCGC
KaM418: CTGCCATTCAGGGGATCCCATATGGAGGAGGGAGACAG

### Beispiel 5

### Klonierung von yaad-Hydrophobin BASF2-His₆

Die Klonierung des Plasmids #506 erfolgte analog zu Plasmid #508 unter Verwendung der Oligonukleotide KaM 417 und KaM 418.

Als Template DNA wurde ein künstlich synthetisierte DNA Sequenz - Hydrophobin BASF2 -eingesetzt (siehe Anhang).
KaM417:CCCGTAGCTAGTGGATCCATTGAAGGCCGCAT-GAAGTTCTCCGTCTCCGC
KaM418: CTGCCATTCAGGGGATCCCATATGGAGGAGGGAGACAG

### Beispiel 6

### Klonierung von yaad-Hydrophobin SC3-His₆

Die Klonierung des Plasmids #526 erfolgte analog zu Plasmid #508 unter Verwendung der Oligonukleotide KaM464 und KaM465.

Als Template DNA wurde cDNA von Schyzophyllum commune eingesetzt (siehe Anhang).
KaM464: CGTTAAGGATCCGAGGATGTTGATGGGGGTGC
KaM465: GCTAACAGATCTATGTTCGCCCGTCTCCCCGTCGT

### Beispiel 7

### Fermentation des rekombinanten E.coli Stammes yaad-Hydrophobin DewA-His₆

Inokulation von 3ml LB Flüssigmedium mit einem yaad-Hydrophobin DewA-His₆ exprimierenden E.coli Stamm in 15ml Greiner Röhrchen. Inkubation für 8h bei 37°C auf einem Schüttler mit 200 UpM. Je 2 1l Erlenmeyer Kolben mit Schikanen und 250ml LB Medium (+ 100µg/ml Ampicillin) werden mit jeweils 1ml der Vorkultur angeimpft und 9h bei 37°C auf einem Schüttler mit 180 UpM inkubiert.
13.51 LB-Medium (+100µg/ml Ampicillin) in einem 20l Fermenter mit 0,5l Vorkultur (OD₆₀₀ₙₘ 1:10 gegen H₂0 gemessen) animpfen. Bei einer OD₆₀ₙₘ von ∼3.5 Zugabe von 140ml 100mM IPTG. Nach 3h Fermenter auf 10°C abkühlen und Fermentationsbrühe abzentrifugieren. Zellpellet zur weiteren Aufreinigung verwenden.

### Beispiel 8

### Reinigung des rekombinanten Hydrohobin-Fusionsproteins

### (Reinigung von Hydrophobin-Fusionsproteinen, die ein C-terminales His6-tag besitzen)

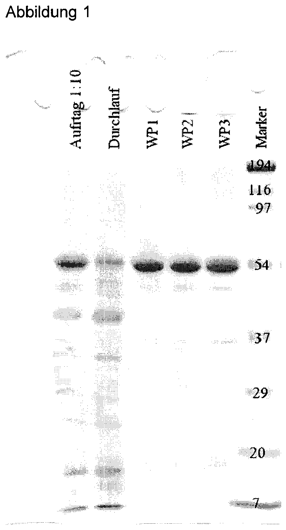

100 g Zellpellet (100 - 500 mg Hydrophobin) werden mit 50 mM Natriumphosphatpuffer, pH 7,5 auf 200 ml Gesamtvolumen aufgefüllt und resuspendiert. Die Suspension wird mit einem Ultraturrax Typ T25 (Janke und Kunkel; IKA-Labortechnik) für 10 Minuten behandelt und anschliessend für 1 Stunde bei Raumtemperatur mit 500 Einheiten Benzonase (Merck, Darmstadt; Best.-Nr. 1.01697.0001) zum Abbau der Nukleinsäuren inkubiert. Vor dem Zellaufschluss wird mit einer Glaskartusche (P1) filtriert. Zum Zellaufschluß und für das Scheren der restlichen genomischen DNA werden zwei Homogenisatorläufe bei 1.500 bar durchgeführt (Microfluidizer M-110EH; Microfluidics Corp.). Das Homogenisat wird zentrifugiert (Sorvall RC-5B, GSA-Rotor, 250 ml Zentrifugenbecher, 60 Minuten, 4°C, 12.000 Upm, 23.000 g), der Überstand auf Eis gestellt und das Pellet in 100 ml Natriumphosphatpuffer, pH 7,5 resuspendiert. Zentrifugation und Resuspendieren werden dreimal wiederholt, wobei der Natriumphosphatpuffer bei der dritten Wiederholung 1 % SDS enthält. Nach der Resuspension wird für eine Stunde gerührt und eine abschliessende Zentrifugation durchgeführt (Sorvall RC-5B, GSA-Rotor, 250 ml Zentrifugenbecher, 60 Minuten, 4°C, 12.000 Upm, 23.000 g). Gemäß SDS-PAGE Analyse ist das Hydrophobin nach der abschliessenden Zentrifugation im Überstand enthalten (Abbildung 1). Die Versuche zeigen, dass das Hydrophobin wahrscheinlich in Form von Einschlusskörpern in den entsprechenden E.coli Zellen enthalten ist. 50 ml des Hydrophobin-enthaltenden Überstandes werden auf eine 50 ml Nikkel-Sepharose High Performance 17-5268-02 Säule aufgetragen (Amersham), die mit 50 mM Tris-Cl pH 8,0 Puffer äquilibriert wurde. Die Säule wird mit 50 mM Tris-Cl pH 8,0 Puffer gewaschen und das Hydrophobin anschliessend mit 50 mM Tris-Cl pH 8,0 Puffer, der 200 mM Imidazol enthält, eluiert. Zur Entfernung des Imidazols wird die Lösung gegen 50 mM Tris-Cl pH 8,0 Puffer dialysiert.

Abbildung 1 zeigt die Reinigung des erfindungsgemässen Hydrophobins:
Spur 1: Auftrag Nickel-Sepharose Säule (1:10 Verdünnung)
Spur 2: Durchlauf = Eluat Waschschritt
Spuren 3 - 5: OD 280 Maxima der Elutionsfraktionen

Das erfindungsgemässe Hydrophobin der Abbildung 1 besitzt ein Molekulargewicht von ca. 53 kD. Die kleineren Banden repräsentieren zum Teil Abbauprodukte des Hydrophobins.

### Beispiel 9

### Beschichtung/Evaluierung von Oberflächen mit Hydrophobin

Die Evaluierung der Beschichtungseigenschaften von Hydrophobin bzw. Hydrophobinfusionsprotein wird bevorzugt auf Glas bzw. Teflon als Modelle für eine hydrophile bzw. hydrophobe Oberflächen vorgenommen.

### Standard-Versuche für Beschichtung

### Glas:

- Konzentration Hydrophobin: 1 - 100 µg/mL
- Inkubation von Glasplättchen über Nacht (Temperatur: 80°C) in 50mM Na-Acetat pH 4 + 0,1 % Tween 20
- nach Beschichtung waschen in VE-Wasser
- danach Inkubation 10min / 80°C / 1% SDS
- waschen in VE-Wasser

### Teflon:

- Konzentration: 1 - 100 µg/mL
- Inkubation von Teflonplättchen über Nacht (Temperatur: 80°C) in 10mM Tris pH 8
- nach Beschichtung waschen in VE-Wasser
- Inkubation 10min / 80°C / 0,1% Tween 20
- waschen in VE-Wasser
- danach Inkubation 10min / 80°C / 1% SDS
- waschen in VE-Wasser

Die Proben werden an der Luft getrocknet und der Kontaktwinkel (in Grad) eines Tropfens von 5 µl Wasser bestimmt. Es ergeben sich z.B. folgende Werte:
Ansatz mit yaad-DewA Fusionsprotein gemäss Beispiel 8(Kontrolle: ohne Protein;
yaad-dewA-his₆: 100 µg/ml gereinigter Fusionspartner):

| | nach 1%SDS 80°C | |
|---|---|---|
| | Teflon | Glas |
| Kontrolle | 96,8 | 30 |
| yaad | 97,4 | 38,7 |
| 100 µg/ml | 77,7 | 76,8 |
| 50 µg/ml | 85,9 | 77,9 |
| 10 µg/ml | 83,5 | 74,5 |
| 5 µg/ml | 104 | 70,3 |
| 1 µg/ml | 104,9 | 73 |

### Beispiel 10

### Beschichtung/Evaluierung von Oberflächen mit Hydrophobin

### Glas (Fensterglas, Süddeutsche Glas, Mannheim):

- Konzentration Hydrophobin: 100 µg/mL
- Inkubation von Glasplättchen über Nacht (Temperatur 80°C) in 50mM Na-Acetat pH 4 + 0,1% Tween 20
- nach Beschichtung waschen in destilliertem Wasser
- danach Inkubation 10min / 80°C / 1% SDS-Lösung in dest. Wasser
- waschen in dest. Wasser

Die Proben werden an der Luft getrocknet und der Kontaktwinkel (in Grad) eines Tropfens von 5 µl Wasser bestimmt.

Die Kontaktwinkelmessung wurde auf einem Gerät Dataphysics Contact Angle System OCA 15+, Software SCA 20.2.0. (November 2002) bestimmt. Die Messung erfolgte gemäss den Herstellerangaben.

Unbehandeltes Glas ergab einen Kontaktwinkel von 30 ± 5°; eine Beschichtung mit einem funktionellen Hydrophobin gemäss Beispiel 8 (yaad-dewA-his₆) ergab Kontaktwinkel von 75 ± 5°.

Zuordnung der Sequenznamen zu DNA- und Polypeptidsequenzen im Sequenzprotokoll

| | |
|---|---|
| dewA DNA- und Polypeptidsequenz | SEQ ID NO: 1 |
| dewA Polypeptidsequenz | SEQ ID NO: 2 |
| rodA DNA- und Polypeptidsequenz | SEQ ID NO: 3 |
| rodA Polypeptidsequenz | SEQ ID NO: 4 |
| hypA DNA- und Polypeptidsequenz | SEQ ID NO: 5 |
| hypA Polypeptidsequenz | SEQ ID NO: 6 |
| hypB DNA- und Polypeptidsequenz | SEQ ID NO: 7 |
| hypB Polypeptidsequenz | SEQ ID NO: 8 |
| sc3 DNA- und Polypeptidsequenz | SEQ ID NO: 9 |
| sc3 Polypeptidsequenz | SEQ ID NO: 10 |
| basf1 DNA- und Polypeptidsequenz | SEQ ID NO: 11 |
| basf1 Polypeptidsequenz | SEQ ID NO: 12 |
| basf2 DNA- und Polypeptidsequenz | SEQ ID NO: 13 |
| basf2 Polypeptidsequenz | SEQ ID NO: 14 |
| yaad DNA- und Polypeptidsequenz | SEQ ID NO: 15 |
| yaad Polypeptidsequenz | SEQ ID NO: 16 |
| yaae DNA- und Polypeptidsequenz | SEQ ID NO: 17 |
| yaae Polypeptidsequenz | SEQ ID NO: 18 |
| yaad-Xa-dewA-his DNA- und Polypeptidsequenz | SEQ ID NO: 19 |
| yaad-Xa-dewA-his Polypeptidsequenz | SEQ ID NO: 20 |
| yaad-Xa-rodA-his DNA- und Polypeptidsequenz | SEQ ID NO: 21 |
| yaad-Xa-rodA-his Polypeptidsequenz | SEQ ID NO: 22 |
| yaad-Xa-basf1-his DNA- und Polypeptidsequenz | SEQ ID NO: 23 |
| yaad-Xa-basf1-his Polypeptidsequenz | SEQ ID NO: 24 |

## Patentansprüche

1. Polypeptide der allgemeinen Strukturformel (I)
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)
wobei X für jede der 20 natürlich vorkommenden Aminosäuren steht,
n und m für Zahlen zwischen 0 und 500 stehen,
C für Cystein steht,
mit der Maßgabe, dass wenigstens eine der mit Xₙ oder Xₘ abgekürzten Peptidsequenzen für eine mindestens 20 Aminosäuren lange Peptidsequenz steht, die natürlicherweise nicht mit einem Hydrophobin verknüpft ist, **dadurch gekennzeichnet, dass** die Struktkurformel (I)
(i) nur ein Hydrophobin umfasst,
(ii) eine Sequenz SEQ ID NO:2 umfasst und
(iii) daß Xₙ oder Xₘ eine Polypeptidsequenz ausgewählt aus SEQ ID NO: 16 oder Fragmenten von SEQ ID NO:16 mit 80-99% der Sequenz umfasst,
die nach Beschichten einer Glasoberfläche eine Kontaktwinkeländerung von mindestens 20° bewirken.

2. Nukleinsäure, codierend für Polypeptide gemäß Anspruch 1

3. Verfahren zur Herstellung von Polypeptiden gemäss Anspruch 1, indem man eine Nukleinsäure gemäss Anspruch 2 in einem Wirtsorganismus exprimiert und das so erhaltene Protein ggf. nach Aufreinigung isoliert.

4. Verfahren nach Anspruch 3, **dadurch gekennzeichnet, dass** als Wirtsorganismus E.coli verwendet wird.

5. Verwendung von Hydrophobinen gemäss Anspruch 1 zur Beschichtung von Oberflächen.

## Claims

1. A polypeptide of the general structural formula (I)
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-X₁₋₅₀-C⁸-Xₘ (I)
where X is any of the 20 naturally occurring amino acids,
n and m are numbers between 0 and 500,
C is cystein,
with the proviso that at least one of the peptide sequences abbreviated as Xₙ or Xₘ is a peptide sequence of at least 20 amino acids in length which is not linked to a hydrophobin naturally, wherein the structural formula (I) comprises
(i) only one hydrophobin,
(ii) a SEQ ID NO: 2 sequence, and
(iii) wherein Xₙ or Xₘ comprises a polypeptide sequence selected from SEQ ID NO: 16 or fragments of SEQ ID NO: 16 with 80-99% of said sequence,
which polypeptide changes the contact angle by at least 20° after coating of a glass surface.

2. A nucleic acid coding for polypeptides as defined in claim 1.

3. A method of producing polypeptides as defined in claim 1 by expressing a nucleic acid as defined in claim 2 in a host organism and isolating the protein thus obtained, optionally after purification.

4. The method according to claim 3, wherein the host organism used is E. coli.

5. The use of hydrophobins as defined in claim 1 for the coating of surfaces.

## Revendications

1. Polypeptides de formule structurale générale (I)
Xₙ-C¹-X₁₋₅₀-C²-X₀₋₅-C³-X₁₋₁₀₀-C⁴-X₁₋₁₀₀-C⁵-X₁₋₅₀-C⁶-X₀₋₅-C⁷-C₁₋₅₀-C⁸-Xₘ (I)
dans laquelle X représente chacun des 20 acides aminés naturels,
n et m représentent des nombres compris entre 0 et 500, C représente la cystéine,
à condition qu'au moins une des séquences peptidiques abrégées par Xₙ ou Xₘ représente une séquence peptidique d'une longueur d'au moins 20 acides aminés, qui n'est pas naturellement reliée avec une hydrophobine, **caractérisés en ce que** la formule structurale (I)
(i) ne comprend qu'une hydrophobine,
(ii) comprend une séquence SEQ ID NO : 2 et
(iii) **en ce que** Xₙ ou Xₘ comprend une séquence polypeptidique choisie parmi SEQ ID NO : 16 ou des fragments de SEQ ID NO : 16 avec 80 à 99 % de la séquence,
qui après le revêtement d'une surface en verre provoquent une modification de l'angle de contact d'au moins 20°.

2. Acide nucléique, codant pour des polypeptides selon la revendication 1.

3. Procédé de fabrication de polypeptides selon la revendication 1, selon lequel un acide nucléique selon la revendication 2 est exprimé dans un organisme hôte et la protéine ainsi obtenue est isolée, éventuellement après purification.

4. Procédé selon la revendication 3, **caractérisé en ce qu'**E. coli est utilisé en tant qu'organisme hôte.

5. Utilisation d'hydrophobines selon la revendication 1 pour le revêtement de surfaces.
